# EUROPEAN PATENT SPECIFICATION

(11) **EP 3 006 078 B1**
(45) Date of publication and mention of the grant of the patent: **07.10.2020**
(21) Application number: 14807088.1
(22) Date of filing: 03.06.2014
(51) Int. Cl.: B29C 43/02, A61M 37/00, B29C 33/42, B29C 39/02, B29C 41/04, B29L 31/00, B29C 43/14, B29C 43/20

(54) **NEEDLE BODY MANUFACTURING METHOD**
VERFAHREN ZUR HERSTELLUNG EINES NADELKÖRPERS
PROCÉDÉ DE FABRICATION DE CORPS D'AIGUILLE

(30) Priority: 03.06.2013 JP 2013116895; 09.01.2014 JP 2014002305; 16.01.2014 JP 2014005547; 28.02.2014 JP 2014038223; 02.05.2014 JP 2014095102; 02.05.2014 JP 2014095103
(43) Date of publication of application: 13.04.2016
(73) Proprietor: Toppan Printing Co., Ltd., Tokyo 110-0016 (JP)
(72) Inventor: KATO, Hiroyuki, Tokyo 110-0016 (JP); TAGAMI, Hanae, Tokyo 110-0016 (JP); UENO, Hisami, Tokyo 110-0016 (JP); GUNJIMA, Seiji, Tokyo 110-0016 (JP); MATSUZAWA, Hiroshi, Tokyo 110-0016 (JP); ARAKI, Momoko, Tokyo 110-0016 (JP); TAKAHATA, Shigeki, Tokyo 110-0016 (JP)
(74) Representative: TBK
(86) International application number: PCT/JP2014/064718
(87) International publication number: WO 2014/196522

(56) References cited:
- EP-A1- 2 921 202
- WO-A2-2010/140760
- WO-A2-2010/140760
- WO-A2-2012/153266
- JP-A- 2009 082 206
- JP-A- 2009 273 772
- US-A1- 2008 275 400
- US-A1- 2008 275 400
- US-A1- 2009 234 301

## Description

### [Technical Field]

The present invention relates to a manufacturing method for an acicular body. More specifically, the present invention relates to a method for manufacturing an acicular body that includes a fine needle and in which the needle pierces skin when the acicular body is adhered to the skin.

### [Background Art]

In a transdermal absorption method, a substance to be delivered is administered into a body by penetration of the substance to be delivered into the body from the skin. The transdermal absorption method enables a substance to be delivered to be easily administered without causing a person pain.

In the field of transdermal administration in which the transdermal absorption method is used, a method is proposed in which a substance to be delivered or the like is administered into the skin by using an acicular body that is provided with a needle in the order of micrometers to pierce the skin. Refer to PTL 1.

As a manufacturing method for the acicular body, preparing an original plate using machine processing, forming a transfer plate from the original plate, and performing transfer processing and molding using the transfer plate has been proposed. Refer to PTL 2.

Furthermore, preparing an original plate using an etching process, forming a transfer plate from the original plate, and performing transfer processing and molding using the transfer plate is proposed. Refer to PTL 3.

The acicular body may become fractured during use, and the fractured acicular body may remain within the body. The material composing the acicular body preferably does not adversely affect the human body. Therefore, a biocompatible material, such as chitin or chitosan, is proposed as an acicular body material. Refer to PTL 4.

### [Citation List]

### [Patent Literature]

[PTL 1] JP-A-S48-93192
[PTL 2] WO 2008/013282
[PTL 3] WO 2008/004597
[PTL 4] WO 2008/020632

US 2008/275400 A1 is directed at the making of microneedle arrays by injection molding of a molten polymeric material onto a carrier web.

JP 2009-273772 A discloses a manufacturing method for an acicular body provided with a support substrate and a needle provided on the support substrate, the manufacturing method comprising:
- a first step of dripping a needle forming solution into a recessed cavity having a shape corresponding to the needle; H
- a second step of disposing a water-soluble resin film on the mould; and
- a third step of absorbing the moisture of the aqueous solution inside the recess to solidify the needle forming solution, and forming the needle.

### [Summary of Invention]

### [Technical Problem]

When the needle of the acicular body is formed of a biocompatible material composed of a water-soluble polymer, the needle can be dissolved inside the skin after piercing the skin. As a result of the needle being fabricated from a material containing a substance to be delivered for the purpose of delivery into the skin, in addition to the water-soluble polymer, a needle that contains the substance to be delivered can be formed. When a needle such as this pierces the skin, the substance can be delivered into the skin in accompaniment with the dissolution of the water-soluble polymer.

To fabricate the needle from a water-soluble polymer material, there is a method in which a water-soluble polymer material solution is cast in an acicular body intaglio plate, and the acicular body intaglio plate is then dried. There is also a method in which the acicular body is obtained through freeze-drying and solidification of the material. A method in which heating at a desired temperature is performed to quicken drying is also known. However, a problem occurs in that, in this method, air remaining within an acicular body recess of the intaglio plate expands, thereby causing mingling of air bubbles in the solution that fills the intaglio plate. As a result, air bubbles remain in the obtained acicular body.

As a method for avoiding the formation of air bubbles during heating and drying, and avoiding the mingling of air bubbles in the acicular body, there is a method in which the intaglio plate is placed in a reduced pressure environment after being supplied with the acicular body material solution. As a result, the recess of the intaglio plate is filled with the material solution, while air bubbles remaining within the recess are removed. The air bubbles within the recess can also be removed in a pressurized environment, instead of a reduced pressure environment.

To remove air bubbles from the recess in a reduced pressure environment, the air bubbles are required to be moved to the top surface of the material solution supplied to the top surface of the recess. Therefore, when a high-viscosity material solution is used, the air bubbles are not easily released. Contrary to intention, the mingling of air bubbles in the acicular body may be promoted.

On the other hand, when the recess is filled with the material in a pressurized environment, pressurization is required to be performed while controlling the pressurization speed such that the acicular body material solution supplied to the recess of the intaglio plate does not move from the desired supply location and the boundary surface does not shake. Since a pressurization chamber is required, a problem of increasing manufacturing cost arises.

In a method for fabricating a multilayer acicular body, such as a two-layer acicular body, the recess of the intaglio plate is filled with a material solution serving as a first layer. The material solution fills the recess as a result of the recess being suctioned from a plate bottom portion in a reduced pressure environment or a pressurized environment. Subsequently, excess material solution is removed, and drying and solidification are performed. Thereafter, a material solution serving as a second layer is supplied to the recess. An operation similar to that for the first layer is performed, thereby enabling the two-layer acicular body to be obtained.

A multilayer acicular body can be obtained by the above-described method. However, when fabricating the two-layer acicular body, for example, a problem occurs in that a material filling step involves two steps and production takt time becomes long.

In addition, excess material is required to be removed after material filling of the first layer is performed. A problem occurs in that material loss increases. Although the material can be reused, the acicular body is configured to be capable of delivering an active ingredient and the like by dissolving within a living body. Therefore, confirmation that the reused material does not affect the living body is required to be made.

An object of the present invention is to provide an acicular body manufacturing method that enables an acicular body to be manufactured at low cost and under atmospheric pressure, while minimizing the mingling of air bubbles.

### [Solution to Problem]

A first aspect of the present invention is a manufacturing method for an acicular body provided with a support substrate and a needle provided on the support substrate, the manufacturing method including: a first step of supplying a needle forming solution containing a needle material that forms the needle onto an intaglio plate having a recess that has a shape corresponding to the needle; a second step of disposing a film on the needle forming solution, compressing the needle forming solution from above the film, and moving the needle forming solution into the recess; and a third step of removing liquid components from the needle forming solution inside the recess, solidifying the needle forming solution, and forming the needle.

In the manufacturing method for an acicular body, the needle forming solution may be heated at the third step.

Another manufacturing method for an acicular body of the present invention is a manufacturing method for an acicular body provided with a support substrate and a needle provided on the support substrate, the manufacturing method including: a first step of supplying water onto an intaglio plate having a recess that has a shape corresponding to the needle; a second step of disposing a film composed of a needle material that forms the needle on the water, compressing the needle forming solution from above the film, and moving the needle material into the recess while dissolving the needle material in the water; and a third step of removing liquid components from the solution containing the needle material that has moved into the recess, solidifying the solution, and forming the needle.

In the manufacturing method for an acicular body, the water may be supplied and the film may be disposed by the water being attached to the film and the film being disposed on the intaglio plate, at the first step and the second step.

Still another manufacturing method for an acicular body of the present invention is a manufacturing method for an acicular body provided with a support substrate and a needle provided on the support substrate, the manufacturing method including: a first step of supplying a needle forming solution containing a needle material that forms the needle onto an intaglio plate having a recess that has a shape corresponding to the needle; a second step of disposing a film composed of a water-soluble material on the needle forming solution, compressing the needle forming solution from above the film, and moving the needle forming solution into the recess; and a third step of removing liquid components from the needle forming solution inside the recess, solidifying the needle forming solution, and forming the needle.

In the manufacturing method for an acicular body, the needle material and the water-soluble material may be the same substance.

### [Advantageous Effects of Invention]

As a result of the manufacturing method for an acicular body of the present invention, an acicular body can be manufactured at low cost and under atmospheric pressure, while minimizing the mingling of air bubbles.

### [Brief Description of Drawings]

Fig. 1 is a schematic diagram of an acicular body that is manufactured according to an embodiment of the present invention;
Fig. 2 is a cross-sectional view of a part of a cross-sectional structure of the acicular body in Fig. 1;
Fig. 3 is a schematic diagram showing an acicular body according to a first embodiment of the present invention in detail;
Fig. 4 is a diagram of a process in a manufacturing method for the same acicular body;
Fig. 5 is a diagram of a process in the manufacturing method for the same acicular body;
Fig. 6 is a diagram of a process in the manufacturing method for the same acicular body;
Fig. 7 is a diagram of a process in the manufacturing method for the same acicular body;
Fig. 8 is a schematic diagram of an acicular body according to a second embodiment of the present invention;
Fig. 9 is a diagram of a process in a manufacturing method for the same acicular body;
Fig. 10 is a diagram of a process in the manufacturing method for the same acicular body;
Fig. 11 is a diagram of a process in the manufacturing method for the same acicular body;
Fig. 12 is a diagram of a process in a manufacturing method of a modification for the same acicular body;
Fig. 13 is a diagram of a process in the manufacturing method of the same modification;
Fig. 14 is a diagram of a process in the manufacturing method of the same modification;
Fig. 15A is a cross-sectional schematic diagram of an acicular body according to a third embodiment of the present invention;
Fig. 15B is a cross-sectional schematic diagram of the acicular body according to the third embodiment of the present invention;
Fig. 15C is a cross-sectional schematic diagram of the acicular body according to the third embodiment of the present invention;
Fig. 16 is a table showing the constituent materials of a needle-side layer and a support substrate-side layer;
Fig. 17A is a diagram for explaining a manufacturing method for a first acicular body;
Fig. 17B is a diagram for explaining the manufacturing method for the first acicular body;
Fig. 17C is a diagram for explaining the manufacturing method for the first acicular body;
Fig. 17D is a diagram for explaining the manufacturing method for the first acicular body;
Fig. 17E is a diagram for explaining the manufacturing method for the first acicular body;
Fig. 17F is a diagram for explaining the manufacturing method for the first acicular body;
Fig. 18A is a diagram for explaining a manufacturing method for a second acicular body;
Fig. 18B is a diagram for explaining the manufacturing method for the second acicular body;
Fig. 18C is a diagram for explaining the manufacturing method for the second acicular body;
Fig. 19A is a diagram for explaining the manufacturing method for the second acicular body;
Fig. 19B is a diagram for explaining the manufacturing method for the second acicular body;
Fig. 19C is a diagram for explaining the manufacturing method for the second acicular body;
Fig. 20A is a diagram for explaining a manufacturing method for a third acicular body;
Fig. 20B is a diagram for explaining the manufacturing method for the third acicular body;
Fig. 20C is a diagram for explaining the manufacturing method for the third acicular body
Fig. 21A is a diagram for explaining the manufacturing method for the third acicular body;
Fig. 21B is a diagram for explaining the manufacturing method for the third acicular body;
Fig. 21C is a diagram for explaining the manufacturing method for the third acicular body;
Fig. 22A is a diagram for explaining the manufacturing method for the third acicular body;
Fig. 22B is a diagram for explaining the manufacturing method for the third acicular body;
Fig. 23A is a diagram for explaining a manufacturing method for a fourth acicular body;
Fig. 23B is a diagram for explaining the manufacturing method for the fourth acicular body;
Fig. 23C is a diagram for explaining the manufacturing method for the fourth acicular body;
Fig. 24A is a diagram for explaining the manufacturing method for the fourth acicular body;
Fig. 24B is a diagram for explaining the manufacturing method for the fourth acicular body;
Fig. 24C is a diagram for explaining the manufacturing method for the fourth acicular body;
Fig. 25A is a diagram for explaining the manufacturing method for the fourth acicular body;
Fig. 25B is a diagram for explaining the manufacturing method for the fourth acicular body;
Fig. 26A is an explanatory diagram of a manufacturing method for an acicular body according to a fourth embodiment of the present invention;
Fig. 26B is an explanatory diagram of the manufacturing method for the acicular body according to the fourth embodiment of the present invention;
Fig. 26C is an explanatory diagram of the manufacturing method for the acicular body according to the fourth embodiment of the present invention;
Fig. 26D is an explanatory diagram of the manufacturing method for the acicular body according to the fourth embodiment of the present invention;
Fig. 26E is an explanatory diagram of the manufacturing method for the acicular body according to the fourth embodiment of the present invention;
Fig. 26F is an explanatory diagram of the manufacturing method for the acicular body according to the fourth embodiment of the present invention;
Fig. 26G is an explanatory diagram of the manufacturing method for the acicular body according to the fourth embodiment of the present invention;
Fig. 27A is an explanatory diagram of a manufacturing method for an acicular body in a modification;
Fig. 27B is an explanatory diagram of a manufacturing method for an acicular body in the modification;
Fig. 27C is an explanatory diagram of a manufacturing method for an acicular body in the modification;
Fig. 27D is an explanatory diagram of a manufacturing method for an acicular body in the modification;
Fig. 28 is a perspective view of a warpage correcting member used in a manufacturing method for an acicular body according to a fifth embodiment of the present invention;
Fig. 29 is a diagram of an intaglio plate in a manufacturing process according to the fifth embodiment of the present invention;
Fig. 30 is a diagram of a filling step in the manufacturing process according to the fifth embodiment of the present invention;
Fig. 31 is a diagram of a drying step in the manufacturing process according to the fifth embodiment of the present invention;
Fig. 32 is a diagram of a releasing step in the manufacturing process according to the fifth embodiment of the present invention;
Fig. 33 is a diagram of a drying step in a manufacturing process according to a sixth embodiment of the present invention;
Fig. 34 is a diagram of an overview of a manufacturing apparatus;
Fig. 35 is a block diagram of an electrical configuration of the manufacturing apparatus in Fig. 34;
Fig. 36 is a diagram of an example of a state where an intaglio plate is set on a stage;
Fig. 37 is a diagram of an example of an operating state of the manufacturing apparatus;
Fig. 38 is a diagram of an overview of a manufacturing apparatus in a modification;
Fig. 39 is an explanatory diagram of a manufacturing method for an acicular body according to an eighth embodiment of the present invention;
Fig. 40 is an explanatory diagram of the manufacturing method for an acicular body according to the eighth embodiment of the present invention;
Fig. 41 is an explanatory diagram of the manufacturing method for an acicular body according to the eighth embodiment of the present invention;
Fig. 42 is an explanatory diagram of the manufacturing method for an acicular body according to the eighth embodiment of the present invention;
Fig. 43 is an explanatory diagram of the manufacturing method for an acicular body according to the eighth embodiment of the present invention;
Fig. 44 is an explanatory diagram of the manufacturing method for an acicular body according to the eighth embodiment of the present invention;
Fig. 45 is an explanatory diagram of the manufacturing method for an acicular body according to the eighth embodiment of the present invention;
Fig. 46 is a table showing the results of examples 1 to 19 and comparative examples 1 to 3;
Fig. 47 show microscopic observation photograms of example 20 and example 21; and
Fig. 48 is a table showing the results of examples 25 and 26 and comparative examples 4 and 5.

### [Description of Embodiments]

### [Configuration of an acicular body]

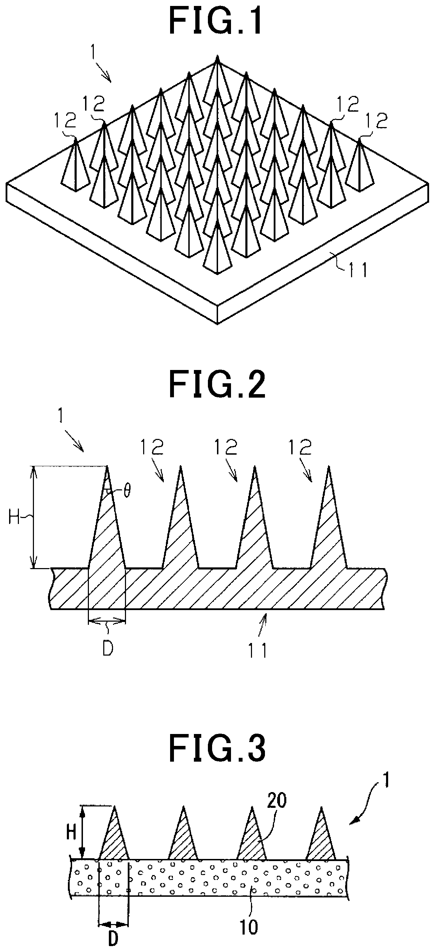

A typical configuration of an acicular body manufactured by a manufacturing method of the present invention will be described with reference to Fig. 1 and Fig. 2.

As shown in Fig. 1, an acicular body 1 includes a plate-shaped substrate 11 and a plurality of protrusion portions 12 that protrude from the front surface of the substrate 11. The bottom surface of the protrusion portion 12 is formed integrally with the front surface of the substrate 11. The substrate 11 supports the base end of each of the plurality of protrusion portions 12.

The shape of the protrusion portion 12 may be a pyramid shape or a circular conical shape. For example, the protrusion portion 12 may have a shape in which the tip is not pointed, such as a circular columnar shape or a prismatic shape. Furthermore, for example, the protrusion portion 12 may have a shape in which two or more solids are coupled, such a shape in which a circular cone is stacked onto a circular column. In short, all that is required is that the protrusion portion 12 have a shape capable of piercing skin. The number of protrusion portions 12 can be optionally determined, or only has to be one or more.

A substance to be delivered inside a target of piercing may be contained in the acicular body 1. The substance to be delivered may be dispersed inside the target of piercing as a result of the protrusion portion 12 dissolving inside the target of piercing. Alternatively, the substance to be delivered may be supplied from outside of the acicular body 1. Whether the substance to be delivered is contained in the acicular body 1 or supplied from outside of the acicular body 1 differs depending on the mode of use of the acicular body 1.

When the substance to be delivered is supplied from outside of the acicular body 1, a hole for passing the substance to be delivered towards the inside of the target of piercing may be formed in the protrusion portion 12. The hole formed in the protrusion portion 12 may be a hole that penetrates from the tip of the protrusion portion 12 to the back surface of the substrate 11, or may be a non-penetrating hole. Regardless of whether or not a hole is provided, a groove that passes the substance towards the inside of the target of piercing may be formed around the protrusion portion 12. A hole that passes the substance supplied from outside of the acicular body 1 towards the protrusion portion 12 may be formed in the substrate 11. The hole formed in the substrate 11 may be a hole that penetrates from the front surface of the substrate 11 to the back surface of the substrate 11, or may be a non-penetrating hole.

The plurality of protrusion portions 12 may be arranged in a regular manner on the front surface of the substrate 11 or may be arranged in an irregular manner. The plurality of protrusion portions 12 may be arranged such as to be localized in a plurality of locations on the front surface of the substrate 11. For example, the plurality of protrusion portions 12 are aligned in an array. Here, "in an array" refers to a state where the needles are arranged in a predetermined form. For example, the array includes a grid array, a closest-packed array, a concentric circular array, and a random array.

The dimensions of the protrusion portion 12 are preferably set to have a narrowness and length suitable for forming a piercing hole in the skin. As shown in Fig. 2, a height H of the protrusion portion 12 is preferably from 10 µm to 1000 µm, both inclusive. The height H of the protrusion portion 12 is the length from the front surface of the substrate 11 to the tip of the protrusion portion 12. The height H of the protrusion portion 12 is determined based on the depth of the hole that is required in the target of piercing. When the target of piercing is the skin on a human body and the bottom of a hole is set within the stratum corneum, the height H is preferably from 10 µm to 300 µm, both inclusive, and more preferably from 30 µm to 200 µm, both inclusive. When the bottom of the hole is set such as to penetrate the stratum corneum to a depth that does not reach the nervous layer, the height H is preferably from 200 µm to 700 µm, both inclusive, more preferably from 200 µm to 500 µm, both inclusive, and still more preferably from 200 µm to 300 µm, both inclusive. When the bottom of the hole is set to a depth that reaches the dermis, the height H is preferably from 200 µm to 500 µm, both inclusive. When the depth of the hole is set to a depth that reaches the epidermis, the height H is preferably from 200 µm to 300 µm, both inclusive.

A width D of the protrusion portion 12 is the maximum value of the length of the protrusion portion 12 in the direction parallel to the front surface of the substrate 11. The width D of the protrusion portion 12 is preferably from 1 µm to 300 µm, both inclusive. For example, when the protrusion portion 12 has a square conical shape or a square columnar shape, the length of the diagonal line of the square defined by the bottom portion of the protrusion portion 12 on the front surface of the substrate 11 corresponds to the width D of the protrusion portion 12. For example, when the protrusion portion 12 has a circular conical shape or a circular columnar shape, the diameter of the circle plotted by the bottom portion of the protrusion portion 12 corresponds to the width D of the protrusion portion 12.

An aspect ratio A (A=H/D), which is the ratio of the height H to the width D of the protrusion portion 12, is preferably from 1 to 10, both inclusive.

When the tip of the protrusion portion 12 is formed into a pointed shape and the hole is formed to a depth that penetrates the stratum corneum, a point angle θ of the protrusion portion 12 is preferably from 5° to 30°, both inclusive, and more preferably from 10° to 20°, both inclusive. The point angle θ refers to the maximum value of the angle formed by the tip of the protrusion portion 12 on a cross-section that is perpendicular to the front surface of the substrate 11. For example, when the protrusion portion 12 has a square conical shape, the point angle θ of the protrusion portion 12 is the vertex angle of a triangle of which the diagonal line of the square plotted by the bottom portion of the protrusion portion 12 is the bottom side and the vertex of the square cone is the vertex.

The width D, the aspect ratio A, and the point angle θ of the protrusion portion 12 are determined based on the capacity and the like required by the hole. As long as the height H, the width D, the aspect ratio A, and the point angle θ are within the above-described ranges, the shape of the protrusion portion 12 is a shape suitable for the shape of a hole formed in the skin.

The acicular body 1 is preferably formed from a material comprising a water-soluble material and a substance to be delivered. When the substance to be delivered is contained in the acicular body 1 in a state where the substance is held in the water-soluble material that functions as a base, and the acicular body 1 is used on a human body, the substance is dispersed within the body as a result of the protrusion portion 12 being dissolved by the moisture in the skin. In the case of a mode in which the water-soluble material functions as a substance to be delivered or a mode in which the substance to be delivered is supplied from outside of the acicular body 1, the acicular body 1 does not have to contain a substance to be delivered separate from the water-soluble material.

When the acicular body 1 is used on the human body, the acicular body 1 is preferably formed from a material having biocompatibility. A water-soluble material having biocompatibility includes water-soluble polymers such as carboxymethyl cellulose (CMC), methylcellulose (MC), hydroxypropyl cellulose (HPC), hydroxypropyl methylcellulose (HPMC), ethyl cellulose, hydroxyethyl cellulose, polyvinyl alcohol (PVA), polyacrylate-based polymers, polyacrylamide (PAM), polyethylene oxide (PEO), pullulan, alginate, starch, pectin, chitosan, chitosan succinamide, oligo chitosan, and sodium chondroitin sulfate. However, the water-soluble polymer in the water-soluble material of the present invention is not limited to specific materials.

A polysaccharide, such as dextran, dextrin, trehalose, and maltose, can be used as the water-soluble material that has biocompatibility. However, the polysaccharide in the water-soluble material of the present invention is not limited to a specific material.

Among the above-described water-soluble polymers, chitosan, chitosan succinamide, hydroxypropyl cellulose (HPC), carboxymethyl cellulose (CMC), and hydroxypropyl methylcellulose (HPMC) have high biological safety, and are therefore particularly preferable as the material for the needle.

Among the above-described polysaccharides, trehalose, maltose, and the like are preferably used. This is because trehalose in particular has a crystalline structure that is particularly close to that of water, even among disaccharides. Therefore, when the substance to be delivered is a protein, trehalose protects and stabilizes the protein.

Even in the case of a material other than the above-described water-soluble materials, if the material can be applied to a manufacturing method in which the acicular body 1 is formed by an intaglio plate being filled with a liquid body containing the material and liquid body being cured by drying, the material can be used as a formation material of the acicular body 1. The water-soluble material of the present invention is not limited to the above-described specific materials.

Various proteins, pharmacologically active substances, cosmetic compositions, and the like can be used as the substance to be delivered. The type of substance to be delivered is selected depending on the purpose.

Pharmacologically active substances include, for example, a vaccine such as that for influenza, a pain-relief drug for cancer patients, a biologic, a gene therapy drug, an injectable agent, an oral agent, or a dermatological preparation. In transdermal administration using the acicular body 1, the substance to be delivered is administered into the hole formed in the skin. Therefore, transdermal administration using the acicular body 1 can also be used for administration of pharmacologically active substances that require subcutaneous injection, in addition to pharmacologically active substances used in conventional transdermal administration. In particular, because transdermal administration using the acicular body 1 is painless during administration, transdermal administration using the acicular body 1 is suitable for administration of injectable agents, such as a vaccine, to children. Since the substance to be delivered is not required to be swallowed during administration, transdermal administration using the acicular body 1 is suitable for administration of an oral agent to children who have difficulty swallowing oral agents.

The cosmetic composition is a composition used as a cosmetic or a beauty product. The cosmetic composition includes, for example, moisturizing agents, coloring materials, fragrances, and physiologically active substances that exhibit beautifying effects such as beneficial effects on wrinkles, acne, stretch marks, and the like, as well as beneficial effects regarding epilation. When a material having fragrance is used as the substance to be delivered, the scent can be imparted to the acicular body 1. Therefore, an acicular body 1 suitable for beauty products is obtained.

The ratio of the substance to be delivered and the water-soluble polymer contained in the acicular body 1 is determined taking into consideration the amount of substance required to be administered into the body, and the amount of water-soluble polymer required for formation of the acicular body 1.

These materials may be referred to, hereafter, as a "needle material", as a collective term.

### [First embodiment]

A first embodiment of the present invention will be described with reference to Fig. 3 to Fig. 7. First, the structure of an acicular body manufactured according to the present embodiment will be described.

The acicular body 1 according to the present embodiment includes a support substrate 10 and a needle 20 formed on the support substrate 10. According to the present embodiment, the support substrate 10 forms the substrate 11. The needle 20 forms the protrusion portion 12.

The support substrate 10 is an air-permeable film composed of a resin, and has air-permeability enabling gas to be transmitted in the thickness direction. The air-permeable film is merely required to allow moisture included in a material aqueous solution of the needle 20 to turn into water vapor and be transmitted and evaporated, during the manufacturing process of the acicular body described hereafter. The material is not particularly limited. For example, a porous film composed of cellophane, polyethylene, polypropylene, polyimide, or the like, a non-woven fabric, or fibers can be used. The air-permeable film may be a cloth that is composed of fibers and has air permeability, such as a mesh used in screen printing. A stainless steel mesh can also be used. Natural fibers, chemical fibers, or metal fibers can be used as the fibers.

In the present invention, the film includes a sheet-shaped article and a plate-shaped article.

The air-permeable film preferably has a moisture permeability of 20 g/m²·hr or more, measured based on "Testing Methods for Water Vapor Permeability of Textiles" JIS-L-1099 (2012). The upper limit value of the moisture permeability of the air-permeable film is not an issue, as long as the film shape can be maintained.

The needle 20 is composed of a material that dissolves after piercing the skin, that is, a water-soluble material that is capable of liquefaction. The desired substance to be delivered is contained in the needle. As a result of the needle dissolving within the skin, the substance is introduced into the body via the inside of the skin.

A manufacturing method for the acicular body 1 that has the above-described configuration will be described.

First, an intaglio plate for forming the needle is prepared. An original plate that determines the shape of each needle 20 is fabricated. An intaglio plate in which the protrusions and recesses of the shape of the original plate are inverted is fabricated. The original plate can be manufactured by a publically known method based on the shape of the needle. The original plate may be formed using a microfabrication technique. The microfabrication technique includes, for example, lithography, wet etching, dry etching, sandblasting, laser machining, and precision machining. A publically known feature replication method can be used to form the intaglio plate from the original plate. The feature replication method includes, for example, formation of a Ni-based intaglio plate by Ni-electrocasting and transfer-molding using a molten resin.

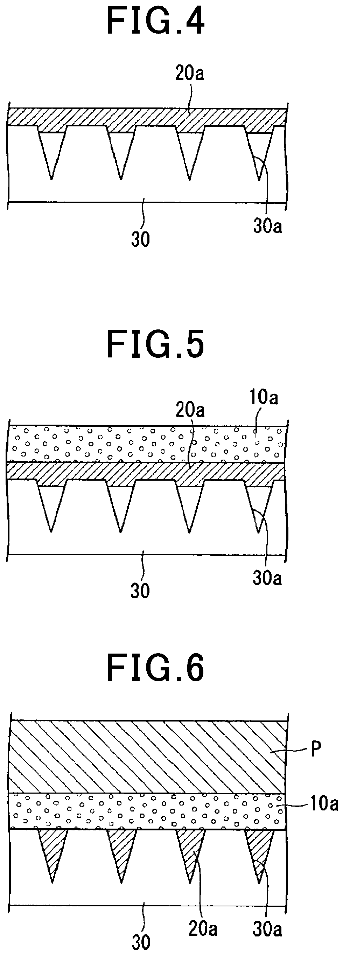

An intaglio plate 30 having a recess 30a that has a shape corresponding to the needle 20 is formed as shown in Fig. 4, by the above-described process.

Next, a needle forming solution containing the needle material and the substance to be delivered is prepared. The fluidity of the needle forming solution is preferably set to a level that enables favorable filling of the recess 30a of the intaglio plate 30 by appropriate adjustment of the amount of solute and the like.

Next, as shown in Fig. 4, a needle forming solution 20a is supplied onto the intaglio plate 30 (first step). The supplying method can be selected as appropriate from publically known methods taking into consideration the shape, dimensions, and the like of the intaglio plate 30. For example, a spin-coat method, a method using a dispenser, a casting method, or an ink-jet method can be used. The supply of needle forming solution 20a to the intaglio plate 30 may be performed under atmospheric pressure. However, to more favorably fill the recess 30a with the needle forming solution 20a, supplying may be performed under reduced pressure or under vacuum. The amount of the needle forming solution 20a is preferably to an extent that all recesses 30a are covered.

Next, as shown in Fig. 5, an air-permeable film 10a is disposed on the needle forming solution 20a as a film serving as the support substrate 10. As shown in Fig. 6, a press member P is placed in contact with a porous film 10a, and the needle forming solution 20a is compressed (second step). The press member P may be roll-shaped, bar-shaped, or bar-shaped with a spherical tip. Various publically known types can be selected and used as appropriate. The material of the press member P can be selected from metal, rubber, resin, and the like.

As a result of the second step, the needle forming solution 20a is compressed and moves into each recess 30a.

Next, the liquid component within the needle forming solution 20a is removed, the needle forming solution 20a is solidified, thereby forming the needle 20 (third step).

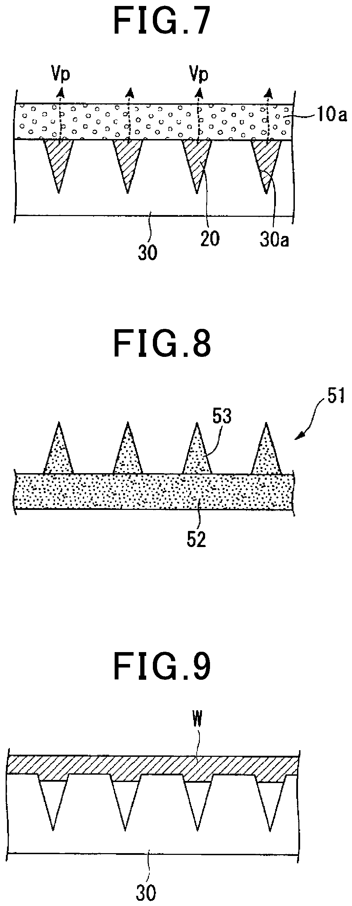

As shown in Fig. 7, the third step can be performed by holding and drying at normal temperature after the press member P is separated from the air-permeable film 10a. Heat-drying of the needle forming solution 20a by heating the intaglio plate 30 or the like is preferable become required time can be shortened.

The liquid component within the needle forming solution 20a can be vaporized and transmitted through the air-permeable film 10a (reference symbol Vp shown in Fig. 7). Therefore, the third step can be performed with the air-permeable film 10a remaining disposed on the needle forming solution 20a. When the needle forming solution 20a is heated, air bubbles may be formed. However, because gas in the air bubbles is also transmitted through the porous film 10a, the air bubbles are favorably removed from within the recess 30a. However, because it is most preferable that air bubbles themselves are not formed, the heating temperature is preferably to an extent that the needle forming solution 20a does not boil, such as within a range from 40°C to 90°C, both inclusive. The heating method is not particularly limited. For example, a hot plate on which the intaglio plate 30 is placed can be used.

After formation of the needle 20, the acicular body 1 including the support substrate 10 and the needle 20 is completed.

The acicular body 1 may be punched into the desired size and shape depending on the intended use and the like, after being peeled from the intaglio plate 30. Punching may be performing using a punching blade, such as a Thomson blade. Alternatively, the air-permeable film 10a can be punched together with the intaglio plate 30, before the air-permeable film 10a is peeled from the intaglio plate 30.

As a result of an adhesive being attached to the periphery of the needle, an acicular body that can be attached to the skin or the like is formed. An adhesive composed of a material that is suitable for attachment to the skin is preferably used. An adhesive capable of withstanding a sterilizing step is more preferable.

When the acicular body 1 is removed from the intaglio plate 30, the acicular body 1 may be peeled from the intaglio plate 30 or the intaglio plate 30 may be chemically dissolved.

According to the above-described first embodiment, the following advantageous effects can be achieved.
(1) At the second step, the needle forming solution 20a is compressed in a state where the air-permeable film 10a is disposed on the needle forming solution 20a. Therefore, the recess 30a can be favorably filled with the needle forming solution 20a. Furthermore, at the third step, the vaporized liquid component of the needle forming solution 20a can be transmitted through the air-permeable film 10a and transpired. Therefore, the needle can be formed by the needle forming solution 20a being favorably dried and solidified, while the air-permeable film 10a remains disposed on the needle forming solution 20a.

As a result, an acicular body can be manufactured at low cost and under atmospheric pressure, while minimizing mingling of air bubbles.

### [Second embodiment]

A second embodiment of the present invention will be described with reference to Fig. 8 to Fig. 14. The material of a support substrate of an acicular body 51 according to the second embodiment differs from that of the acicular body 1 according to the first embodiment. Configurations in the description hereafter that are the same as those already described are given the same reference numbers. Redundant descriptions are omitted.

Fig. 8 is a schematic diagram of the acicular body 51. The acicular body 51 includes a support substrate 52 and a needle 53 formed on the support substrate 52. The support substrate 52 and the needle 53 are composed of the same water-soluble material (referred to, hereafter, as a "water-soluble material A") that is a needle material. A water-soluble polymer or a polysaccharide can be used as the water-soluble material A.

The manufacturing method for the acicular body 51 is substantially similar to that according to the first embodiment. As the needle forming solution, a needle forming solution containing the above-described water-soluble material A is used. At a second step, a film composed of the water-soluble material A is disposed on the needle forming solution.

At a third step, unlike that according to the first embodiment, the liquid component in the needle forming solution is removed by being absorbed by the film composed of the water-soluble material A. The mass of the film composed of the water-soluble material A is significantly greater than the mass of the needle. Therefore, even when the film composed of the water-soluble material A absorbs some of the liquid component, such as the moisture within the needle forming solution, the film composed of the water-soluble material A does not completely dissolve. The sheet-like shape can be retained. Ultimately, the liquid component absorbed by the film composed of the water-soluble material A is also removed from the film composed of the water-soluble material A by transpiration or the like.

In the manufacturing method for an acicular body according to the second embodiment as well, an acicular body can be manufactured at low cost and under atmospheric pressure, while minimizing mingling of air bubbles, in a manner similar to (1) according to the first embodiment.

A modification according to the present embodiment will be described with reference to Fig. 9 to Fig. 11.

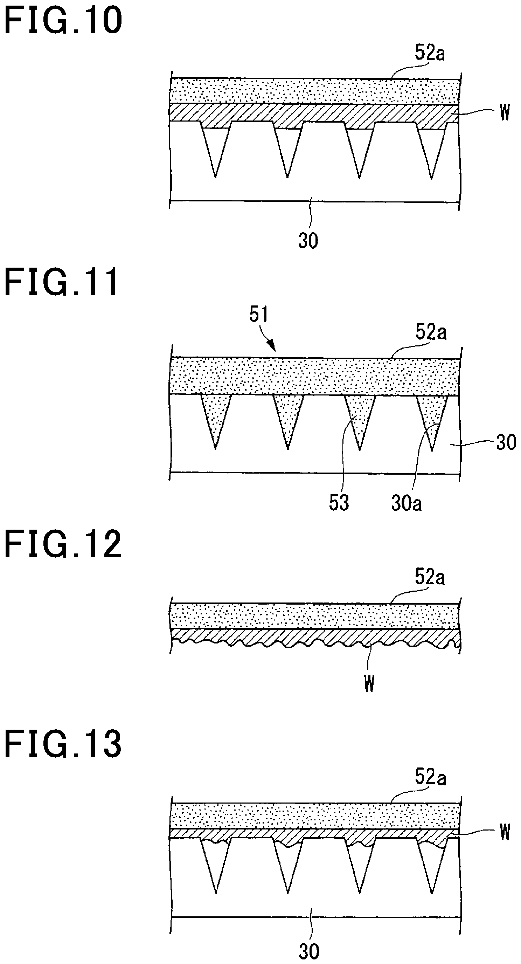

At a first step shown in Fig. 9, water W is supplied onto the intaglio plate 30. As shown in Fig. 10, at a second step, a film 52a composed of the water-soluble material A is placed and compressed.

At a third step, a portion of the water-soluble material A of the film 52a is dissolved in the water W that has moved into the recess 30a. As a result, the water W is gradually removed from within the recess 30a as the water-soluble material A moves into the recess 30a. As shown in Fig. 11, when the third step is completed, the acicular body 51 including the needle 53 composed of the water-soluble material A is completed. In the modification, a portion of the water-soluble material A contained in the film 52a moves into the recess 30a and forms the needle 53. Therefore, the thickness of the film 52a and the like are preferably determined taking into consideration the amount of reduction attributed to the movement.

In the modification, the substance to be delivered may be dissolved in the water W or may be placed such as by being applied to the needle that has been formed.

Furthermore, when the recess 30a having an inner surface that has been subjected to a hydrophilic treatment is used, water is more easily disposed inside the recess 30a when the water is supplied onto the intaglio plate 30.

Another modification of the present invention will be described with reference to Fig. 12 to Fig. 14.

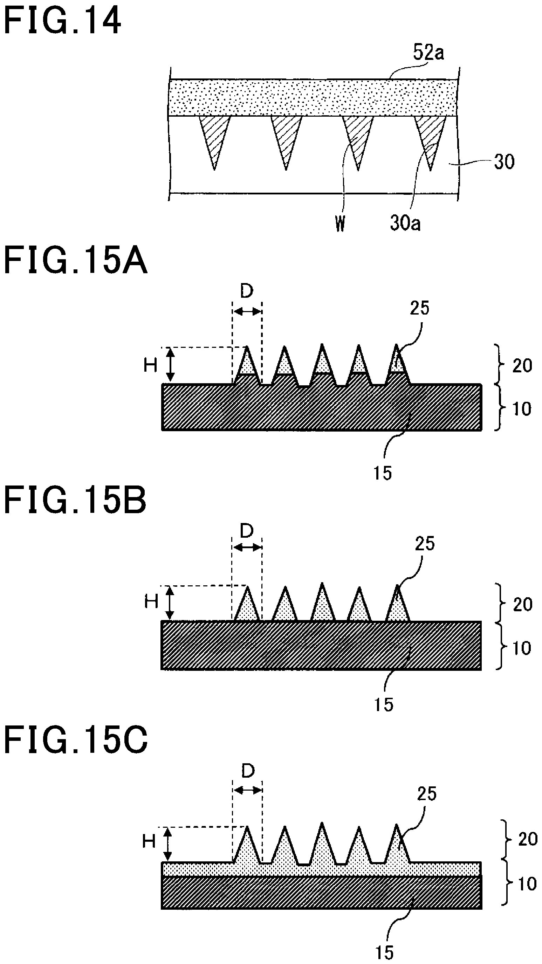

At a first step, one surface of the film 52a is wetted with water W as shown in Fig. 12. As shown in Fig. 13, the film 52a is disposed on the intaglio plate 30 such that the surface wetted with the water W is in contact with the intaglio plate 30. When compressed at a second step, as shown in Fig. 14, the water-soluble material A moves into the recess 30a while being dissolved by the water W attached to the film 52a, and is compressed.

In the modification, because the water W is attached to one surface of the film 52a, the water W is not required to be supplied to match on the position of the recess 30a. Water can be disposed in the recess 30a simply by the film being disposed such as to cover the recess 30a.

In the modification, one surface of the film may be wetted with the needle forming solution.

According to the present embodiment, the water-soluble material forming the film is not necessarily required to be a needle material. In this case, a sufficient amount of the needle material is preferably dissolved in the needle forming solution so that the material of the film does not move to the needle as a result of the above-described solute movement.

The water-soluble material forming the film may be a needle material differing from the needle material forming the needle. In this case, although the needle is composed of two types of needle materials as a result of the movement of the solute, because both materials are needle materials, this is not a significant problem for use.

According to the first and second embodiments, each needle is not required to be independent in the acicular body of the present invention. The needles may be formed such that the lower portions on the support substrate side are connected in a layer shape.

The film used at the second step is not necessarily required to serve as the support substrate. In this case, after needle formation, the film may be moved in parallel with the intaglio plate and removed from the needle. A separate support substrate on which an adhesive layer or the like is formed may be attached to the intaglio plate. As a result, the needle may be attached to the support substrate.

Furthermore, the second step may be performed in a state where a separate film is further disposed on the air-permeable film or the water-soluble material film and covers over to the side surfaces of the intaglio plate. Leakage of the needle forming solution may thereby be prevented. The film used at this time may be an air-permeable film or a water-soluble material film, or a film that is neither. When a film that is neither an air-permeable film nor a water-soluble material film is used, the film may be removed at the third step.

### [Third embodiment]

A third embodiment of the present invention will be described with reference to Fig. 15 to Fig. 25. An acicular body according to the third embodiment differs from the acicular body 1 according to the first embodiment in terms of having two or more layers containing a substance to be delivered in the thickness direction. Configurations described hereafter that are the same as those already described are given the same reference numbers. Redundant descriptions are omitted.

As shown in Fig. 15A to Fig. 15C, the acicular body includes at least two layers that are, from the needle side, a needle-side layer 25 and a support substrate-side layer 15.

At this time, the boundary of the needle-side layer 25 with the support substrate-side layer 15 may be within the needle 20 (Fig. 15A), at the boundary between the needle 20 and the support substrate 10 (Fig. 15B), or within the support substrate 10 (Fig. 15C).

The needle-side layer 25 is formed by a needle-side layer forming liquid 25a being solidified. The support substrate-side layer 15 is formed by a film being attached to the needle-side layer by compression.

The needle-side layer 25 includes at least a binder. The support substrate-side layer 15 includes a binder and a substance to be delivered. The binder contained in the needle-side layer 25 is composed of a liquefiable material.

At least the support substrate-side layer 15 contains the substance to be delivered. The substance is introduced into the skin surface as a result of the support substrate-side layer 15 being dissolved on the skin surface.

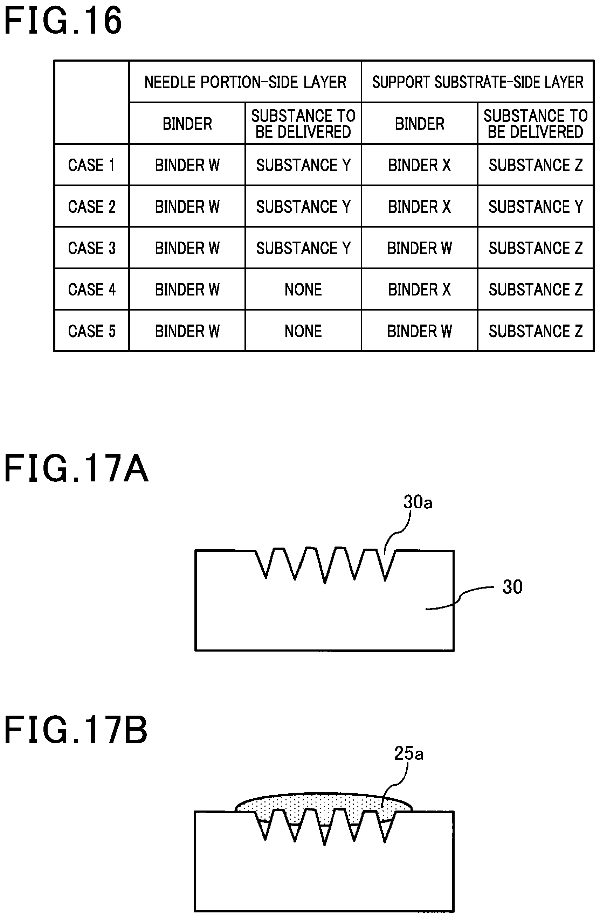

When the constituent materials differ between the needle-side layer 25 and the support substrate-side layer 15, modes shown in Fig. 16 can be considered. Binders W and X represent different materials. Substances Y and Z to be delivered represent different substances.

In the acicular body in case 1, the binder in the needle-side layer is different from the binder in the support substrate-side layer. The substance to be delivered in the needle-side layer is different from the substance to be delivered in the support substrate-side layer.

In the acicular body in case 2, the binder in the needle-side layer is different from the binder in the support substrate-side layer. The substance to be delivered in the needle-side layer is the same as the substance to be delivered in the support substrate-side layer.

In the acicular body in case 3, the binder in the needle-side layer is the same as the binder in the support substrate-side layer. The substance to be delivered in the needle-side layer is different from the substance to be delivered in the support substrate-side layer.

In the acicular body in case 4, the binder in the needle-side layer is different from the binder in the support substrate-side layer. The needle-side layer does not contain a substance to be delivered and only the support substrate-side layer contains a substance to be delivered.

In the acicular body in case 5, the binder in the needle-side layer is the same as the binder in the support substrate-side layer. The needle-side layer does not contain a substance to be delivered and only the support substrate-side layer contains a substance to be delivered.

As mentioned above, the needle-side layer 25 is formed by the needle-side layer forming liquid 25a being solidified. The binder contained in the needle-side layer 25 is composed of a liquefiable material. The liquefiable material includes, as described above, water-soluble materials.

The support substrate-side layer 15 is formed by a film being attached to the needle-side layer 25 by compression. The support substrate-side layer 15 contains the substance to be delivered.

As the film that is the binder used to form the support substrate-side layer 15, a water-soluble material formed into a film can be used. Specifically, one or more can be selected from the above-described water-soluble materials. The film can be fabricated from a water-soluble material liquid using a casting method, a spray-coat method, a spin-coat method, or the like. As a result of the substance to be delivered and the like being dissolved in the water-soluble material liquid in advance, the substance and the like can be contained in the film.

A non-woven fabric, a porous film, or an air-permeable film, namely fibers, can be used as the film that is a binder used when forming the support substrate-side layer 15. Natural fibers, chemical fibers, or metal fibers can be used as the fibers. These films can also be made to contain a substance to be delivered in advance by impregnation or coating.

The substance to be delivered contained in the acicular body includes various proteins, pharmacologically active substances, cosmetic compositions, and the like. The substance to be delivered may be contained in at least the support substrate-side layer 15, and may also be contained in the needle-side layer 25.

A drug that is delivered into the deep part of the skin and exhibits beneficial effects against wrinkles and on immunity is preferably contained in the needle-side layer 25. A drug that exhibits beneficial effects on the skin surface, such as a moisturizing ingredient, is preferably contained in the support substrate-side layer 15.

Next, a manufacturing method for the acicular body according to the present embodiment will be described.

### (Manufacturing method for a first acicular body)

As shown in Fig. 17A, the intaglio plate 30 that has the recess 30a of a shape corresponding to each needle is formed by a process similar to that according to the first embodiment.

Next, the needle-side layer forming liquid 25a is prepared. The fluidity of the needle-side layer forming liquid 25a is preferably set to a level that enables favorable filling of the recess 30a of the intaglio plate 30 by appropriate adjustment of the amount of solute and the like. The needle-side layer forming liquid 25a is fabricated by a binder material, such as a water-soluble polymer or a disaccharide, being dissolved or dispersed in an aqueous solvent, such as water or alcohol. A substance to be delivered is contained in the needle-side formation liquid as required.

Next, as shown in Fig. 17B, the needle-side layer forming liquid 25 is supplied onto the intaglio plate 30 by a process similar to that according to the first embodiment (needle-side layer forming liquid supplying step).

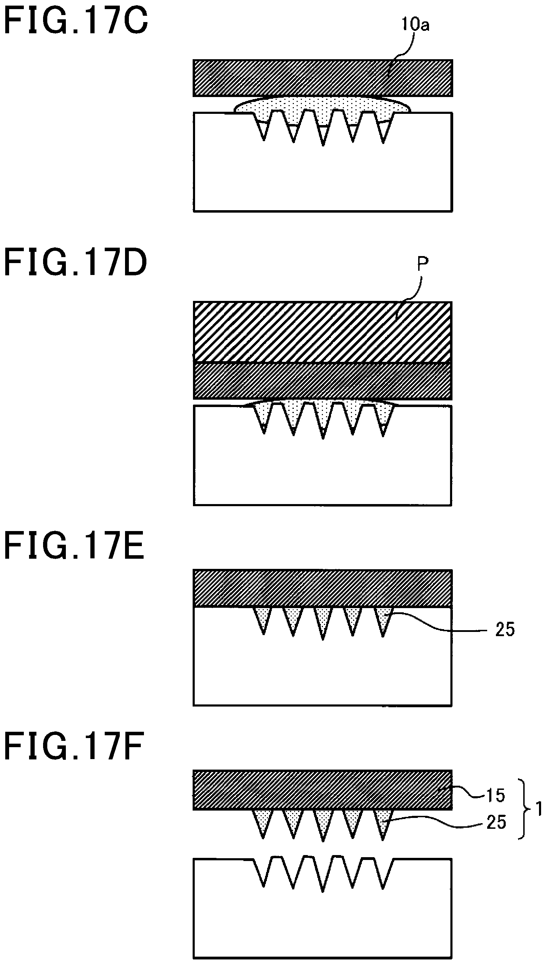

Next, a film 10a, serving as the support substrate 10, is disposed on the needle-side layer forming liquid 25a by a process similar to that according to the first embodiment (Fig. 17C). The press member P is placed in contact with the film 10a and the film 10a is compressed (Fig. 17D) (compressing step).

As a result of the compressing step, the needle-side layer forming liquid 25a is compressed and moves into each recess 30a.

Next, the liquid component within the needle-side layer forming liquid 25a is removed, the needle-side layer forming liquid 25a is solidified, and the needle-side layer 25 is formed (Fig. 17E) (solidifying step).

At the solidifying step, the solvent within the needle-side layer forming liquid 25a is partly absorbed by the film. As a result, removal of the solvent progresses.

When a film composed of a water-soluble material is used, the mass of the film composed of a water-soluble material is significantly greater than the mass of the needle. Therefore, even when some of the liquid component, such as the moisture within the needle-side layer forming liquid 25a is absorbed, the film does not completely dissolve. The sheet-like shape can be retained. Ultimately, the liquid component absorbed by the film is also removed from the film by transpiration or the like. The solidifying step can be performed by holding and drying at normal temperature after the press member P is separated from the film 10a. However, heat-drying of the needle-side layer forming liquid 25a by heating the intaglio plate 30 is preferable become required time can be shortened.

If a non-woven fabric, a porous film, or fibers are used as the film, the solvent within the needle-side layer forming liquid 25a is removed by being absorbed by the film.

The liquid component within the needle-side layer forming liquid 25a can be absorbed by the film and also transmitted. Therefore, the solidifying step can be performed with the film 10a remaining in place.

After formation of the needle-side layer 25, the acicular body 1 including the support substrate-side layer 15 and the needle-side layer 25 is completed. Finally, the acicular body 1 is manufactured by the acicular body being peeled from the intaglio plate 30 (Fig. 17F) (solidifying step).

As described above, the acicular body 1 may be punched into the desired size and shape depending on the intended use and the like, before being peeled from the intaglio plate 30 or after being peeled from the intaglio plate 30. When the acicular body 1 is removed from the intaglio plate 30, the acicular body 1 may be peeled from the intaglio plate 30 or the intaglio plate 30 may be chemically dissolved.

### [Manufacturing method for a second acicular body]

Next, a manufacturing method for a second acicular body will be described. A description of sections that overlap the description of the manufacturing method for the first acicular body is omitted.

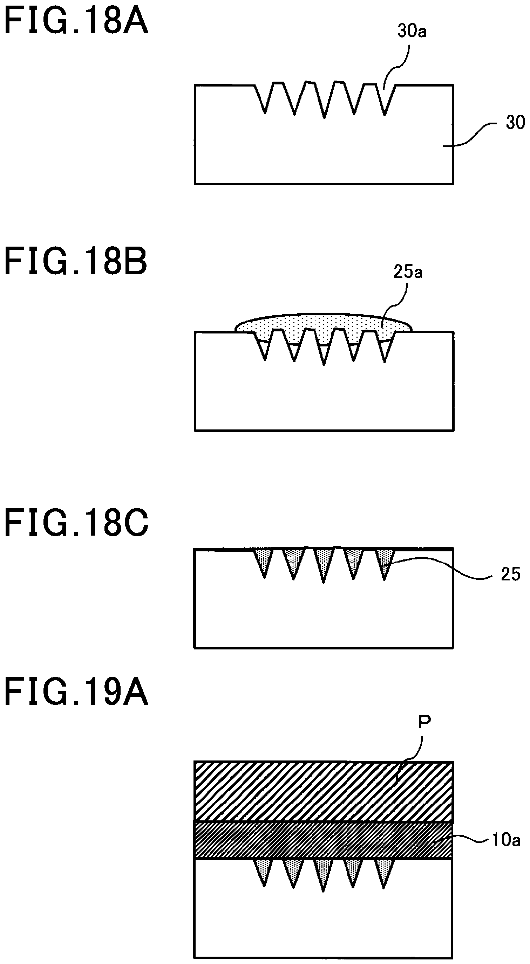

First, an intaglio plate for forming the needle is prepared. An original plate determining the shape of each needle 20 is fabricated, and an intaglio plate in which the protrusions and recesses of the shape of the original plate are inverted is fabricated (Fig. 18A). The needle-side layer forming liquid 25a that contains the needle-side layer formation material, as well as a substance to be delivered as needed, is prepared.

Next, the needle-side layer forming liquid 25a is supplied onto the intaglio plate 30 (Fig. 18B) (needle-side layer forming liquid supplying step).

Next, the liquid component within the needle-side layer forming liquid 25a is removed, solidification is performed, and the needle-side layer 25 is formed (Fig. 18C) (solidifying step). At this time, unlike the manufacturing method for the first acicular body, the intaglio plate 30 is required to be filled with the needle-side layer forming liquid 25a without use of the pressing step. Therefore, a means, such as use of a low-viscosity needle-side layer forming liquid, filling by squeezing after a high-viscosity needle-side layer forming liquid is disposed, or filling with a needle-side layer forming liquid in a vacuum, is required to be used.

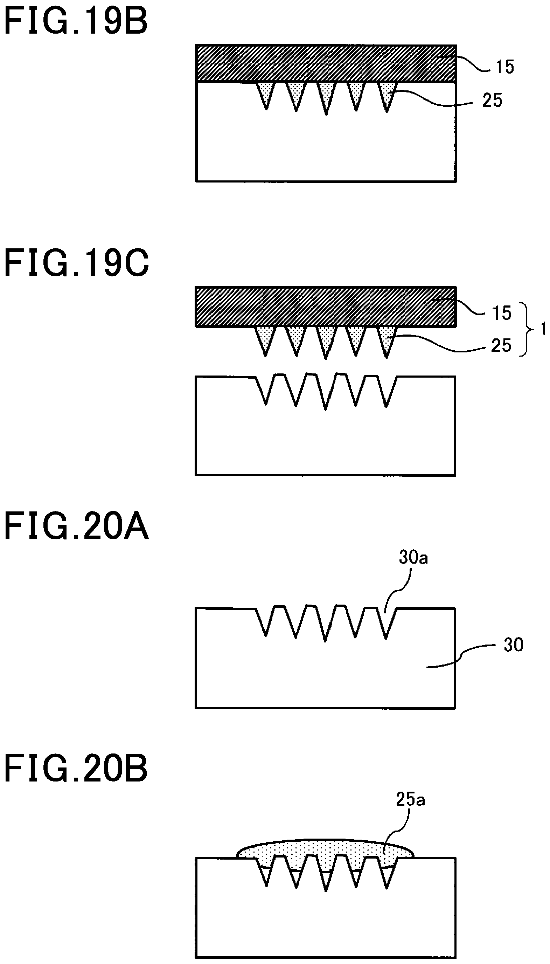

Next, the film 10a containing a substance to be delivered is disposed on the solidified needle-side layer, and the film 10a is compressed (Fig. 19A). As a result, the needle-side layer and the film are bonded (Fig. 19B).

Here, before the film is supplied to the solidified needle-side layer, moisture may be supplied to the surface of the solidified needle-side layer 25. The film may then be disposed and compressed.

As a result, the acicular body 1 including the support substrate-side layer 15 and the needle-side layer 25 is completed. Finally, the acicular body 1 is manufactured by the acicular body being peeled from the intaglio plate 30 (Fig. 19C) (solidifying step).

The acicular body 1 may be punched into the desired size and shape depending on the intended use and the like, before being peeled from the intaglio plate 30 or after being peeled from the intaglio plate 30.

### (Manufacturing method for a third acicular body)

Next, a manufacturing method for a third acicular body will be described. A description of sections that overlap the description of the manufacturing method for the first acicular body and the manufacturing method for the second acicular body is omitted.

First, an intaglio plate for forming the needle is prepared (Fig. 20A). The needle-side layer forming liquid 25a that contains the needle-side layer formation material, as well as a substance to be delivered as needed, is prepared.

Next, the needle-side layer forming liquid 25a is supplied onto the intaglio plate 30 (Fig. 20B) (needle-side layer forming liquid supplying step).

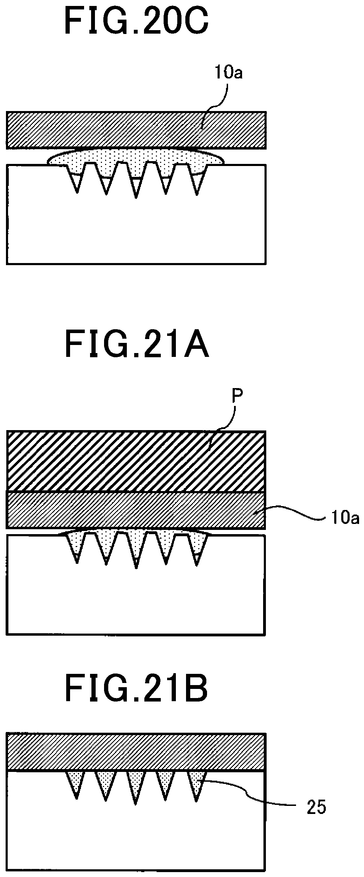

Next, the film 10a, serving as the support substrate 10, is disposed on the needle-side layer forming liquid 25a (Fig. 20C). The press member P is placed in contact with the film 10a, and the film 10a is compressed (Fig. 21A) (compressing step). As a result of the compressing step, the needle-side layer forming liquid 25a is compressed and moves into each recess 30a.

Next, the liquid component within the needle-side layer forming liquid 25a is removed, the needle-side layer forming liquid 25a is solidified, and the needle-side layer 25 is formed (Fig. 21B) (solidifying step). Next, a film is disposed on the solidified needle, and the film is compressed.

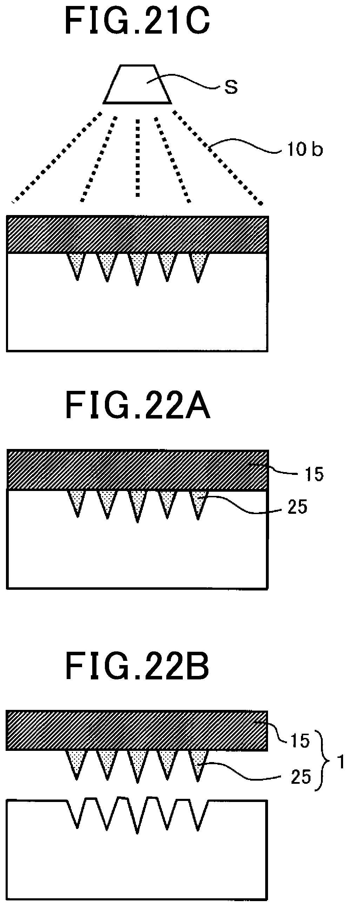

Next, the substance to be delivered is supplied to the film surface (Fig. 21C). As the supply method, a method in which a substance liquid 10b that is dissolved or dispersed in a solvent is sprayed by a spray method, or a method in which the substance liquid 10b is applied by a dispenser method can be performed.

The substance liquid 10b is supplied to the 10A surface on the intaglio plate as a liquid substance, and subsequently dried (Fig. 22A).

As a result, the acicular body 1 that includes the support substrate 10 and the needle 20 is completed. Finally, the acicular body 1 is manufactured by the acicular body being peeled from the intaglio plate 30 (Fig. 22B) (solidifying step).

The acicular body 1 may be punched into the desired size and shape depending on the intended use and the like, before being peeled from the intaglio plate 30 or after being peeled from the intaglio plate 30.

### (Manufacturing method for a fourth acicular body)

Next, a manufacturing method for a third acicular body will be described. A description of sections that overlap the description of the manufacturing method for the first acicular body, the manufacturing method for the second acicular body, and the manufacturing method for the third acicular body is omitted.

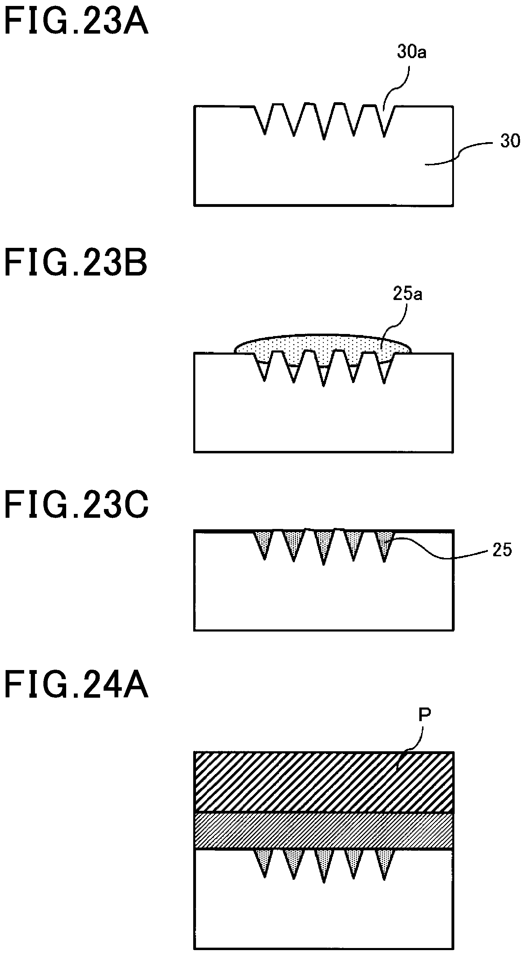

First, an intaglio plate for forming the needle is prepared. An original plate determining the shape of each needle 20 is fabricated, and an intaglio plate in which the protrusions and recesses of the shape of the original plate are inverted is fabricated (Fig. 23A). The needle-side layer forming liquid 25a that contains the needle-side layer formation material, as well as a substance to be delivered as needed, is prepared.

Next, the needle-side layer forming liquid 25a is supplied onto the intaglio plate 30 (Fig. 23B) (needle-side layer forming liquid supplying step).

Next, the liquid component within the needle-side layer forming liquid 25a is removed, the needle-side layer forming liquid 25a is solidified, and the needle-side layer 25 is formed (Fig. 23C) (solidifying step).

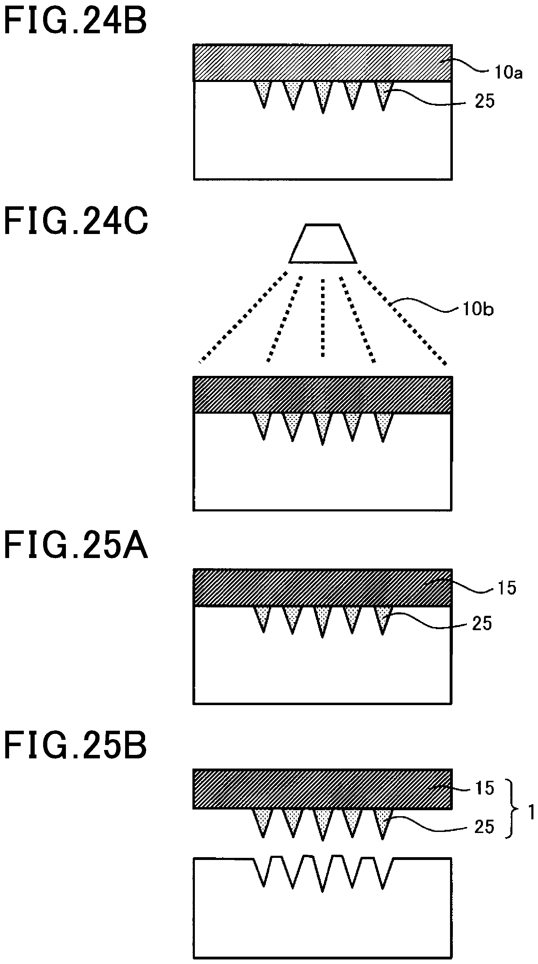

Next, the film 10a is disposed on the solidified needle, and the film is compressed (Fig. 24A). As a result, the needle-side layer 25 and the film 10a are bonded (Fig. 24B).

Next, a substance to be delivered is supplied to the film surface (Fig. 24C).

A substance liquid is supplied to the film surface on the intaglio plate as a liquid substance, and subsequently dried (Fig. 25A).

As a result, the acicular body 1 that includes the support substrate 10 and the needle 20 is completed. Finally, the acicular body 1 is manufactured by the acicular body being peeled from the intaglio plate 30 (Fig. 25B) (solidifying step).

The acicular body 1 may be punched into the desired size and shape depending on the intended use and the like, before being peeled from the intaglio plate 30 or after being peeled from the intaglio plate 30.

The manufacturing methods for the first to fourth acicular bodies of the present invention share a common feature of using a film for the support substrate-side layer. As a result of the film being used, the two-layer acicular body can be more easily manufactured compared to when the acicular body is formed using liquid substances for both layers.

To fabricate the needle from a water-soluble material, there is a method in which a water-soluble material liquid is cast in an acicular body intaglio plate, and subsequently dried. There is also a method in which the acicular body is obtained through freeze-drying and solidification of the material. A method in which heating at a desired temperature is performed to quicken drying is also known. However, a problem occurs in that air remaining within an acicular body recess of the intaglio plate expands, thereby causing mingling of air bubbles in the liquid that fills the intaglio plate. As a result, air bubbles remain in the acicular body.

As a method for avoiding the formation of air bubbles that are formed during heating and drying, and the mingling of air bubbles in the acicular body, there is a method in which the intaglio plate is placed in a reduced pressure environment after being supplied the acicular body material liquid. As a result, the recess of the intaglio plate is filled with the material liquid, while air bubbles remaining within the recess are removed. The air bubbles within the recess can also be removed in a pressurized environment, instead of a reduced pressure environment.

To remove air bubbles from the recess in a reduced pressure environment, the air bubbles are required to be moved to the top surface of the material liquid supplied to the top surface of the recess. Therefore, when a high-viscosity material liquid is used, the air bubbles are not easily released. Contrarily, the mingling of air bubbles in the acicular body may be promoted.

Meanwhile, when the recess is filled with the material in a pressurized environment, pressurization is required to be performed while controlling the pressurization speed such that the acicular body material liquid supplied to the recess of the intaglio plate does not move from the desired supply location and the boundary surface does not shake. Since a pressurization chamber is required, a problem of increasing manufacturing cost arises.

According to the above-described third embodiment, the following advantageous effects can be achieved.
(2) Through use of the film, a manufacturing method for an acicular body that has little mingling of air bubbles can be achieved.
(3) Through use of the film, a manufacturing method for an acicular body having a short production takt time (production time) and little material loss can be achieved.

### [Fourth embodiment]

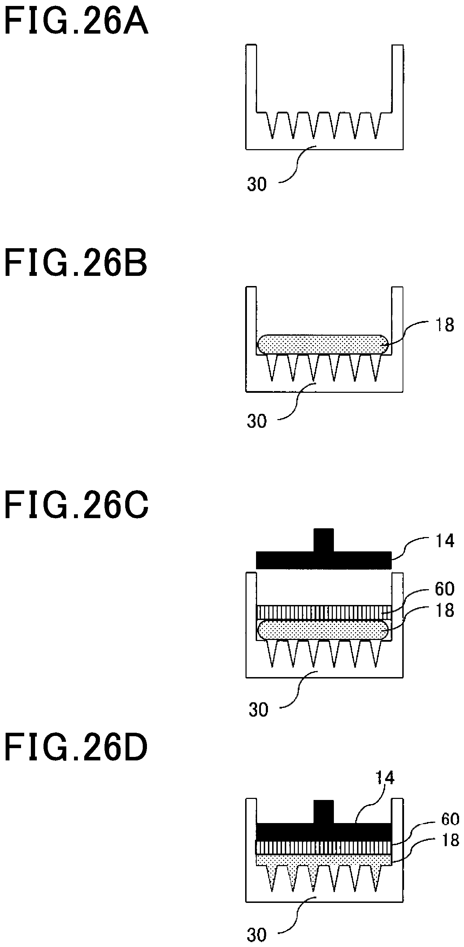

A fourth embodiment of the present invention will be described with reference to Fig. 26A to Fig. 26G. A manufacturing method according to the fourth embodiment differs from the manufacturing method according to the first embodiment in that, in particular, an air-permeable film that is silicone-coated or fluororesin-coated is disposed on the acicular body material solution, to stabilize the shape of the protrusion portion tip of the acicular body. Configurations in the description hereafter that are the same as those already described are given the same reference numbers. Redundant descriptions are omitted.

### <Step of filling with an acicular body material liquid>

As shown in Fig. 26B, an acicular body material liquid 18 is supplied to the intaglio plate 30 having an acicular pattern shown in Fig. 26A.

### <Step of pressing via an air-permeable film>

Next, as shown in Fig. 26C, an air-permeable film 60 is placed on the acicular body material liquid 18. As shown in Fig. 26D, the acicular body material liquid 18 is pressed from above the air-permeable film 60 and fills the acicular pattern portion of the intaglio plate 30, described above.

The air-permeable film is preferably coated to improve releasability. For example, the air-permeable film includes a fibrous film that is silicone-coated or fluororesin-coated. When pressing is performed via a film such as this, the acicular body material liquid does not attach to a pressing mechanism section. Therefore, there is an advantage in which cleaning after acicular body manufacturing is no longer required. Furthermore, as a result of a release coating being applied, the air-permeable film can be easily peeled from the integrated acicular body after drying is completed. When the release coating is not applied, the acicular body and the air-permeable film become integrated after drying.

The air-permeable film preferably has a moisture permeability of 20 g/m²·hr or more and 1000 g/m₂·hr or less, measured based on "Testing Methods for Water Vapor Permeability of Textiles" JIS-L-1099 (2012). For example, the air-permeable film includes fibrous films and silicone films. As a result of an air-permeable film such as this being used, at the subsequent drying step, drying can be performed without evaporation of the solvent accompanying drying being inhibited. When the moisture permeability is less than 20 g/m²·hr, the speed at which the solvent vapor of the acicular body material liquid, which is generated with drying, passes through the air-permeable film becomes low, and drying cannot be performed. When the moisture permeability is higher than 1000 g/m²·hr, the acicular body material liquid easily permeates into the air-permeable film, and peeling of the air-permeable film from the acicular body after the completion of drying becomes difficult.

When paper is used as the air-permeable film, the density of the air-permeable film is preferably 1 g/cm³ or more. When the density is less than 1 g/cm³, the acicular body material liquid easily seeps into the air-permeable film, and the pressing section may become soiled. The density of the paper used as the air-permeable film is preferably set such that the moisture permeability ranges from 20 g/m²·hr to 1000 g/m²·hr.

The material of the press portion nay be selected as appropriate from materials such as metal-based, rubber-based, and plastic-based. The pressing surface may be flat, or may be a curved surface, or may deform in accompaniment with pressing. Pressing may be performed by a roller-like member being rolled. Pressing force may be selected as appropriate based on the material of the pressing portion and pressing method. Heating may be performed during pressing.

### <Step of drying the acicular body material>

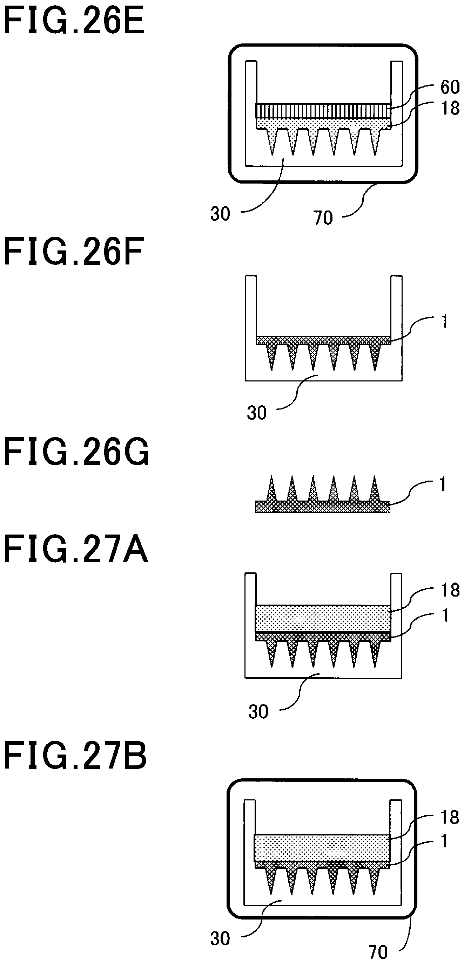

Next, drying and solidification are performed (Fig. 26E). The drying method, such as natural drying, bottom surface heating using a hot plate, or hot-air drying by an oven 70 or the like, may be selected as appropriate based on the environment. However, drying is required to be performed at a temperature at which the aqueous solution does not boil. Drying is preferably performed at a temperature of 110°C or below.

The drying temperature is required to be adjusted based on the moisture permeability of the air-permeable film. For example, when the solvent of the acicular body material liquid is water and the drying temperature is 70°C or higher and no higher than 110°C, the moisture permeability of the air-permeable film preferably ranges from 900 g/m²·hr to 1000 g/m²·hr. When the drying temperature is 50°C or higher and below 70°C, the moisture permeability of the air-permeable film preferably ranges from 300 g/m²·hr to 1000 g/m²·hr. When the drying temperature is 40°C or higher and below 50°C, the moisture permeability of the air-permeable film preferably ranges from 20 g/m²·hr to 1000 g/m²·hr.

### <Step of peeling and removing the air-permeable film>

The above-described air-permeable film is peeled and removed after the completion of drying (Fig. 26F). As a result of the air-permeable film being subjected to release coating, peeling and removal can be easily performed.

### <Step of removing acicular body from the intaglio plate>

Finally, the acicular body 1 can be obtained by being peeled from the intaglio plate 30 (Fig. 26G).

According to the above-described fourth embodiment, the following effect can be achieved.

(4) As a result of the air-permeable film being subjected to release coating, peeling and removal can be easily performed.

When the thickness of the substrate portion of the acicular body 1 after drying is thin, as shown in Fig. 27A, an acicular body material liquid 18 can be additionally supplied. At this time, the type of acicular body material liquid can be changed. In this case, the acicular body that is manufactured can be a two-layered type.

The step of fabricating the intaglio plate is similar to that shown in Fig. 26A.

The step of filling with the acicular body material liquid is similar to that shown in Fig. 26B.

The step of pressing via the air-permeable film is similar to that shown in Fig. 26C and Fig. 26D.

The step of drying the acicular body material is similar to that shown in Fig. 26E.

The step of removing the air-permeable film is similar to that shown in Fig. 26F.

### <Step of drying the added acicular body material>

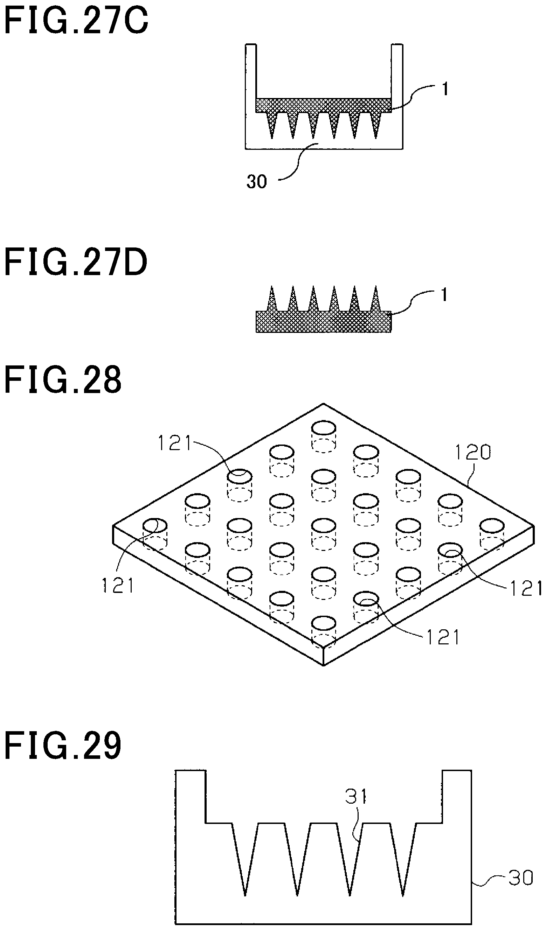

Next, as shown in Fig. 27B and Fig. 27C, the added acicular body material is dried and solidified. The drying method, such as natural drying, bottom surface heating using a hot plate, or hot-air drying, may be selected as appropriate based on the environment. However, the drying is required to be performed at a temperature at which the aqueous solution does not boil. The drying is preferably performed at a temperature of 110° or below.

### <Step of peeling the acicular body from the intaglio plate>

Finally, the acicular body 1 can be obtained by being peeled from the intaglio plate 30 (Fig. 27D).

### [Fifth embodiment]

A fifth embodiment of the present invention will be described with reference to Fig. 28 to Fig. 32. A manufacturing method according to the fifth embodiment differs from that according to the first embodiment in that, in particular, a warpage correcting member is used to minimize the occurrence of warpage at the drying step.

In the manufacturing method for an acicular body of the present invention, the film described according to the first embodiment can also act as a warpage correcting member that corrects warpage of the acicular body during drying. According to the fifth embodiment and a sixth embodiment, described hereafter, of the present invention, the film described according to the first embodiment serves as the warpage correcting member.

Configurations in the description hereafter that are the same as those already described are given the same reference numbers. Redundant descriptions are omitted.

### [Configuration of the warpage correcting member]

A configuration of the warpage correcting member used in the manufacturing method for an acicular body will be described with reference to Fig. 28.

As shown in Fig. 28, a warpage correcting member 120 is a member that extends along a single plane. An air vent 121 that allows gas to pass between the front surface and the back surface of the warpage correcting member 120 is provided.

In the example shown in Fig. 28, the warpage correcting member 120 is formed into a plate shape. The air vent 121 is a through hole that penetrates the warpage correcting member 120 in the thickness direction. The quantity, shape, size, and arrangement of the air vent 121 is not particularly limited. However, the air vent 121 functions as a path for releasing vaporized solvent when the liquid body containing the formation material of the acicular body 1 is dried in the manufacturing process of the acicular body 1. Therefore, the air vent 121 is preferably set to a quantity, shape, size, and arrangement capable of efficiently releasing the vaporized solvent.

The material of the warpage correcting member 120 is not particularly limited. However, for example, a metal, such as aluminum, stainless steel, or iron, is used. As the warpage correcting member 120, for example, a plate composed of such metal that is a metal plate in which a through hole is formed by a punching process, or a metal plate in which through holes are formed in a mesh state by laser machining is used.

Other than the example shown in Fig. 28, the above-described air-permeable film can be used as the warpage correcting member 120. Furthermore, a stainless steel mesh can also be used as the warpage correcting member 120. In these cases, the space between fibers serves as the air vent 121.

The warpage correcting member 120 may be subjected to a water repellent treatment by the surface disposed opposing the top surface of the liquid body being coated with a water repellent material such as a fluorine material or a silicone material. As a result of the warpage correcting member 120 of which the surface opposing the top surface of the liquid body is subjected to water repellent treatment being used, peeling between a molded article and the warpage correcting member 120 after drying can be easily performed.

In particular, when the warpage correcting member 120 is placed in contact on the top surface of the liquid body before the start of drying, the warpage correcting member 120 that is subjected to water repellent treatment is preferably used. At this time, adhesion between the molded article and the warpage correcting member 120 after drying can be prevented.

The water repellent treatment can also be performed by a water repellent material being contained in the natural fibers, chemical fibers, or metal fibers themselves composing the warpage correcting member 120. As the water repellent treatment, a method in which a nickel-fluorine plating treatment is performed on metal fibers can be used. As the fluorine material, tetrafluoroethylene or the like can be used. As the silicone material, a silicone resin or the like can be used.

The warpage correcting member 120 may be a porous film. In short, the warpage correcting member 120 is merely required to be a member that extends along a single plane, and a member that includes the air vent 121 that allows gas to pass through between the front surface and the back surface of the warpage correcting member 120.

### [Manufacturing method for the acicular body]

A manufacturing method for the acicular body will be described with reference to Fig. 29 to Fig. 32. In Fig. 29 to Fig. 32, the number of protrusion portions 12 provided in the acicular body 1 is abbreviated. In the present embodiment, the liquid body includes states from the liquid state before drying is started up to a state of reduced fluidity or a state of partial curing immediately before drying is completed.

### <Intaglio plate fabricating step>

As shown in Fig. 29, at the intaglio plate fabricating step, the intaglio plate 30 having the recess 31 matching the shape of the acicular body 1 is fabricated.

The intaglio plate 30 has the recess 31 of the shape of the acicular body 1 oriented such that the tip of the protrusion portion 12 of the acicular body 1 is disposed on the bottom portion side of the intaglio plate 30 and the back surface of the substrate 11 of the acicular body 1 is disposed on the opening side of the intaglio plate 30.

### <Filling step>

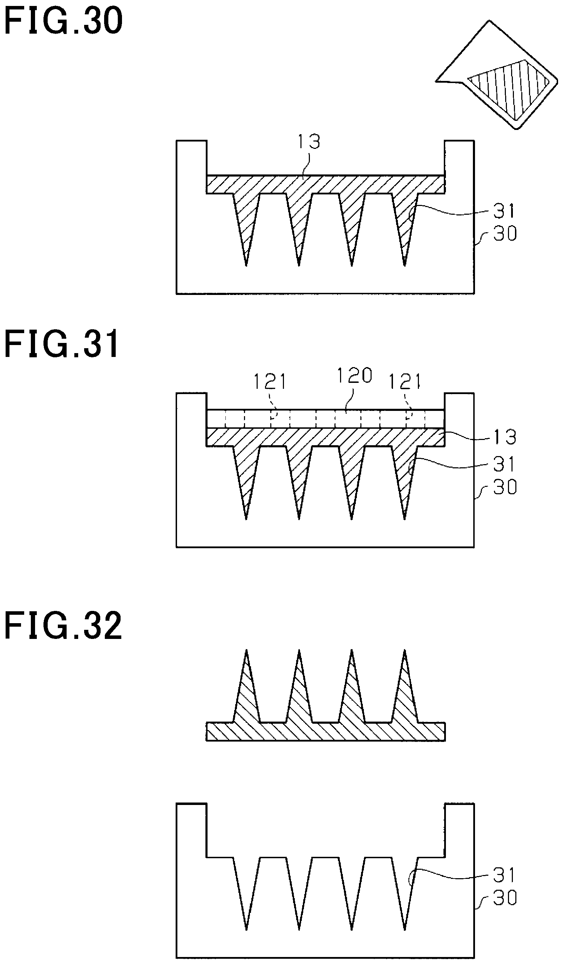

As shown in Fig. 30, at the filling step, the intaglio plate 30 is filled with a liquid body containing the formation material of the acicular body 1. The liquid body is prepared by the formation material of the acicular body 1 being dissolved in a solvent. The type of solvent is not limited as long as the solvent is capable of dissolving the formation material. For example, when the main ingredient of the formation material is a water-soluble material, water is used as the solvent. The liquid body may be a dispersion-based liquid. The liquid body is only required to have fluidity at a level enabling injection into the intaglio plate 30.

The liquid body is injected into the intaglio plate 30 by a method based on the size of the intaglio plate 30 and the shape of the recess 31. As a result, a filling substance composed of the liquid body 13 that fills the inside of the recess 30 is formed.

### <Drying step>

As shown in Fig. 31, at the drying step, the liquid body 13 filling the intaglio plate 30 is dried. As a result, the liquid body 13 is cured and a molded article is formed.

The liquid body 13 may be dried at normal temperature or may be dried in a heated environment. When the liquid body 13 is dried while being heated, the time required for the drying step can be shortened. The heating temperature is preferably a temperature at a degree that the liquid body 13 does not boil, in order to minimize the formation of air bubbles in the acicular body 1. Specifically, when the liquid body 13 is an aqueous solution, the heating temperature is preferably from 50°C to 90°C, both inclusive. A publically known heating method, such as a hot plate, can be used as the heating method.

Here, at the drying step, the above-described warpage correcting member 120 is disposed in the upper portion of the liquid body 13 that fills the intaglio plate 30. The warpage correcting member 120 comes into contact with the top surface of the liquid body 13, which is the surface facing the opening of the intaglio plate 30. In other words, the warpage correcting member 120 comes into contact with the surface that serves as the back surface of the substrate 11 of the acicular body 1 when the liquid body 13 is cured and the acicular body 1 is formed. The warpage correcting member 120 extends along the top surface of the liquid body 13, and covers the overall top surface of the liquid body 13, in sections excluding the air vent 121. In a state where the warpage correcting member 120 is disposed on the top surface of the liquid body 13, the air vent 121 of the warpage correcting member 120 passes gas from below to above the warpage correcting member 120.

When the warpage correcting member 120 has rigidity at a level at which the warpage correcting member 120 does not deform with the warpage of the liquid body 13, such as when the warpage correcting member 120 is a metal plate, the warpage correcting member 120 may be placed on the top surface of the liquid body 13. Alternatively, the periphery of the warpage correcting member 120 may be supported by a supporting member, thereby fixing the warpage correcting member 120 to a position in contact with the top surface of the liquid body 13.

When the rigidity of the warpage correcting member 120 is low, such as when the warpage correcting member 120 is a fabric, the warpage correcting member 120 is fixed to a position in contact with the top surface of the liquid body 13, in a state where the periphery of the warpage correcting member 120 is fixed and tension is applied to the warpage correcting member 120 to an extent that the warpage correcting member 120 does not deform with the warpage of the liquid body 13.

When the warpage correcting member 120 is placed on the top surface of the liquid body 13, the warpage correcting member 120 is preferably disposed on the top surface of the liquid body 13 after drying of the liquid body 13 has started and the liquid body 13 has hardened to an extent that the warpage correcting member 120 does not sink into the liquid body 13, or after a film is formed on the top surface of the liquid body 13.

When the position of the warpage correcting member 120 is fixed, the warpage correcting member 120 may be disposed on the top surface of the liquid body 13 before drying is started or may be disposed on the top surface of the liquid body 13 after drying is started, as long as warpage of the liquid body 13 caused by drying has not yet occurred.

In general, as the liquid body 13 dries, the liquid body 13 shrinks, and the end portion in the direction parallel to the top surface of the liquid body 13 is warped and lifted up towards the opening side of the intaglio plate 30. Regarding this point, as a result of the warpage correcting member 120 being placed, the top surface of the liquid body 13 is pressed by the warpage correcting member 120. Therefore, the occurrence of warpage in the liquid body 13 at the drying step can be minimized. Even when the warpage correcting member 120 is in contact with the liquid body 13, because the air vent 121 is formed in the warpage correcting member 120, the solvent vaporizes and is released outside from the air vent 121. Therefore, the warpage correcting member 120 hardly hampers drying of the liquid body 13 by is minimized.

In a state where curing of the liquid body 13 has been completed and the molded article is molded, the warpage correcting member 120 is in contact with the overall top surface of the molded article, in sections excluding the air vent 121. A part of the top surface of the liquid body 13 may separate from the warpage correcting member 120 as a result of shrinking of the liquid body 13. However, in this case as well, warpage of the liquid body 13 to an extent that the top surface of the liquid body 13 is lifted above the position before the start of drying is minimized.

### <Releasing step>

As shown in Fig. 32, at the releasing step, the warpage correcting member 120 is removed from the molded article that has been molded. The molded article is separated from the intaglio plate 30. The molded article that has been released from the intaglio plate 30 is the acicular body 1. As the method for releasing the molded article, for example, a method in which the molded article is physically peeled from the intaglio plate 30, or a method in which the intaglio plate 30 is selectively dissolved using chemical properties can be used. After the molded article is separated from the intaglio plate 30, the warpage correcting member 120 may be removed.

The acicular body 1 is manufactured by the above-described steps.

As described above, according to the fifth embodiment, the following advantageous effects are achieved.
(5) At the drying step, the top surface of the liquid body 13 is pressed by the warpage correcting member 120. Therefore, occurrence of warpage in the liquid body 13 can be minimized. In other words, the acicular body 1 in which the occurrence of warpage is minimized can be manufactured. The air vent 121 that allows vaporized solvent to pass is formed in the warpage correcting member 120. Therefore, the warpage correcting member 120 hardly hampers drying of the liquid body 13.
   When the occurrence of warpage is minimized, in the manufacturing process, the accuracy of transfer of the shape of the recess 31 in the intaglio plate 30 to the acicular body can be increased. When a film or the like is attached to the back surface of the substrate 11 during storage or during use of the acicular body 1, adhesion between such film and the acicular body 1 is improved as a result of the warpage of the acicular body 1 being minimized.
(6) The warpage correcting member 120 is disposed in a position in contact with the top surface of the liquid body 13 at the start of the period during which the warpage correcting member 120 is disposed on the top surface of the liquid body 13. Therefore, the liquid body 13 warpage and lifting above the position of the liquid body 13 before the warpage correcting member 120 is disposed is minimized. Therefore, warpage that occurs at the drying step is appropriately minimized.
(7) When the warpage correcting member 120 is composed of metal or fibers, the warpage correcting member 120 that functions appropriately is obtained by easily available materials.
(8) When the acicular body 1 is formed from a material containing a water-soluble polymer, warpage more easily occurs at the drying process particularly as the molecular weight of the polymer increases. Therefore, when the above-described manufacturing method is used as the manufacturing method for an acicular body (microneedle) that contains a water-soluble polymer, the effects thereof can be prominently achieved.

### [Sixth embodiment]

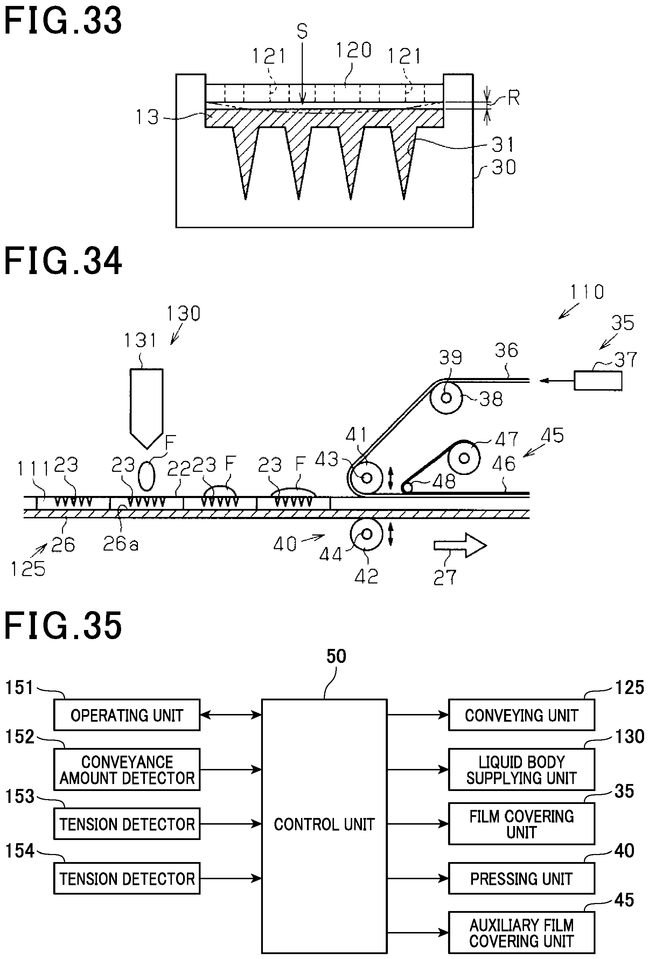

A sixth embodiment of a manufacturing method for an acicular body will be described with reference to Fig. 33.

According to the sixth embodiment, the mode of placement of a warpage correcting member at the drying step differs, compared to the fifth embodiment. Hereafter, the differences with the fifth embodiment will mainly be described. Configurations that are similar to those according to the fifth embodiment are given the same reference numbers. Descriptions thereof are omitted.

### [Manufacturing method for an acicular body]

According to the sixth embodiment, after steps similar to the intaglio plate fabricating step and the filling step according to the fifth embodiment are performed, the filling substance composed of the liquid body 13 that fills the inside of the intaglio plate 30 is formed.

### <Drying step>

As shown in Fig. 33, at the drying step, as a result of the liquid body 13 that fills the intaglio plate 30 being dried, the liquid body 13 is cured and a molded article is formed. As the conditions related to the drying environment, conditions similar to the conditions given as examples at the drying step according to the fifth embodiment are applied.

At the drying step, the warpage correcting member 120 that is described according to the fifth embodiment is disposed on the upper portion of the liquid body 13 that fills the intaglio plate 30. The warpage correcting member 120 is disposed in a position away from the top surface of the liquid body 13. A space S is formed between the liquid body 13 and the warpage correcting member 120. The warpage correcting member 120 extends along the top surface of the liquid body 13 and covers the overall top surface of the liquid body 13, in sections excluding the air vent 121.

When the amount of lifting, caused by warpage of the liquid body 13, of the top surface of the liquid body 13 from the position before the start of drying is a lifting amount R, a space S is set to a size that is the allowed lifting amount R or less. The lifting amount R is the distance between the position of the top surface of the liquid body 13 before drying is started and the position of a section closest to the opening of the intaglio plate 30 on the top surface of the molded article after drying is completed, in the depth direction of the intaglio plate 30. In Fig. 33, the top surface of the molded article after drying is completed is indicated by a two-dot chain line.

When the warpage correcting member 120 has a rigidity at a level at which the warpage correcting member 120 does not deform with the warpage of the liquid body 13, such as when the warpage correcting member 120 is a metal plate, as a result of the periphery of the warpage correcting member 120 being supported by a supporting member, the warpage correcting member 120 is fixed to a position separated from the top surface of the liquid body 13 by the space S.

When the rigidity of the warpage correcting member 120 is low, such as when the warpage correcting member 120 is a fabric, the warpage correcting member 120 is fixed to a position separated from the top surface of the liquid body 13 by the space S, in a state where the periphery of the warpage correcting member 120 is fixed and tension is applied to the warpage correcting member 120 to an extent that the warpage correcting member 120 does not deform with the warpage of the liquid body 13.

The warpage correcting member 120 may be disposed in the upper portion of the liquid body 13 before drying is started or may be disposed in the upper portion of the liquid body 13 after drying is started, as long as warpage of the liquid body 13 caused by drying has not yet occurred.

At the drying step, when the liquid body 13 starts to warp, the top surface of the liquid body 13 comes into contact with the warpage correcting member 120, and the top surface of the liquid body 13 is pressed by the warpage correcting member 120. Therefore, the lifting amount R can be kept to the size of the space S. As a result, the lifting amount R can be reduced to an allowable amount or lower, and the occurrence of warpage in the liquid body 13 that exceeds the allowable amount at the drying step can be minimized.

Even when the warpage correcting member 120 covers the top surface of the liquid body 13, because the air vent 121 that passes gas from below to above the warpage correcting member 120 is formed in the warpage correcting member 120, the solvent vaporizes and is released outside from the air vent 121. Therefore, the warpage correcting member 120 hardly hampers drying of the liquid body 13.

In a state where curing of the liquid body 13 has been completed and the molded article is molded, the warpage correcting member 120 is in contact with the section of the top surface of the molded article that has been lifted from the position before drying is started and reached the position of the warpage correcting member 120. That is, the warpage correcting member 120 is in contact with a portion of the top surface of the molded article.

When drying of the liquid body 13 is completed and the molded article is molded, in a manner similar to the releasing step according to the fifth embodiment, the warpage correcting member 120 is removed from the molded article and the molded article is separated from the intaglio plate 30. At this time, compared to that according to the fifth embodiment, according to the sixth embodiment, the area over which the top surface of the molded article and the warpage correcting member 120 are in contact is small. Therefore, removal of the warpage correcting member 120 is facilitated.

The acicular body 1 is manufactured by the above-described steps.

As described above, according to the sixth embodiment, in addition to the advantageous effects of (5), (7), and (8) according to the fifth embodiment, the following effect is achieved.

(9) The warpage correcting member 120 is disposed in a position away from the top surface of the liquid body 13 at the start of the period during which the warpage correcting member 120 is disposed on the top surface of the liquid body 13. As a result, compared to when the warpage correcting member 120 is disposed in a position in contact with the top surface of the liquid body 13, because the area over which the molded article and the warpage correcting member 120 are in contact is small, removal of the warpage correcting member 120 can be facilitated. Since space is formed above the top surface of the liquid body 13 during drying, the drying of the liquid body 13 can easily progress.

The space S is merely required to be a size that is the allowed lifting amount R or lower. However, at least a portion of the molded article being in contact with the warpage correcting member 120 indicates that the function provided by the warpage correcting member 120 to minimize warpage of the liquid body 13 is being exhibited.

### (Modifications)

The above-described fifth embodiment and sixth embodiment can be carried out with the following modifications.
- After the intaglio plate 30 is filled with the liquid body 13, a film member separate from the warpage correcting member may be supplied to the top surface of the liquid body 13 that fills the intaglio plate 30. The film member configures the acicular body 1 integrally with the liquid body 13. As the film member, a water-soluble material film composed of a water-soluble material in the same manner as the liquid body 13 can be used. Furthermore, as the film member, a water-soluble material film composed of the same type of water-soluble polymer as the liquid body 13 is preferably used. As a result of the film member being used, the time required for the filling substance to dry can be shortened.

When the film member is used, at the filling step, first, the intaglio plate 30 is filled with the liquid body 13. Next, the film member is disposed on the top surface of the liquid body 13 such as to cover the top surface of the liquid body 13. Then, the film member is pressed towards the bottom portion of the intaglio plate 30 from above the film member. As a result, a filling substance composed of the liquid body 13 and the film member is formed. At the filling step, the filling substance may be formed by the warpage correcting member 120 being disposed on the top surface of the film member disposed on the top surface of the liquid body 13, and pressing being performed from above the warpage correcting member 120.

As a result of the above-described configuration as well, when the function for minimizing the warpage of the filling substance provided by the warpage correcting member 120 is exhibited, at a least a portion of the section cured by drying of the filling substance is in contact with the warpage correcting member 120.
- The warpage correcting member 120 does not have to be removed, and for example, may be used as a supporting body that supports the acicular body 1 from the back surface of the substrate 11 during storage or during use of the acicular body 1.
- The warpage correcting member 120 does not necessarily have to cover the overall top surface of the liquid body 13. For example, the warpage correcting member 120 may be disposed in the upper portion in an end portion in which warpage of the liquid body 13 tends to occur.
- The warpage correcting member 120 may be removed from the upper portion of the liquid body 13 before drying of the liquid body 13 is completed and the liquid body 13 is completely cured. In short, at the drying step, the period during which the liquid body 13 is drying is merely required to include a period during which the warpage correcting member 120 is disposed in the upper portion of the liquid body 13. In this case as well, when at least a portion of the section that has been cured by drying in the liquid body 13 is in contact with the warpage correcting member 120, during the period in which the warpage correcting member 120 is disposed in the upper portion of the liquid body 13, the function for minimizing the warpage of the liquid body 13 provided by the warpage correcting member 120 is exhibited.

### (Manufacturing apparatus for the acicular body 1)

A manufacturing apparatus for an acicular body that manufactures the above-described acicular body 1 will be described with reference to Fig. 34 to Fig. 37.

As shown in Fig. 34, a manufacturing apparatus 110 includes a conveying unit 125 that conveys an intaglio plate 111 as a moving unit that configures a de-airing unit.

The intaglio plate 111 has a planar recess forming surface 22. A recess pattern composed of a plurality of recesses 23 is formed in the recess forming surface 22. The recess 23 models the protrusion portion 12. A single recess pattern may be formed in the intaglio plate 111. Alternatively, a plurality of recess patterns may be formed, each recess pattern corresponding to an individual acicular body 1. The intaglio plate 111 is fabricated by a heated relief-plate original plate being pressed onto a plate-shaped base material composed of polyethylene. The relief-plate original plate is thereafter peeled from the base material. The relief-plate original plate is preferably fabricated by cut-machining a metal material, such as a copper-plated metal, brass, nickel, or aluminum. When a plurality of recess patterns are formed in the intaglio plate 111, the relief-plate original plate may be configured such that the plurality of recess patterns are simultaneously formed in the base material. Alternatively, the relief-plate original plate may be configured such that the plurality of recess patterns are formed by the base material being pressed at different positions on the base material.

The conveying unit 125 includes a stage 26 on which the intaglio plate 111 is set. The intaglio plate 111 is set on the stage 26 by being fixed to a setting surface 26a of the stage 26. At the same time, the intaglio plate 111 is set such that the direction in which the recess forming surfaces 22 are aligned runs along the conveying direction 27 of the stage 26. The conveying unit 125 conveys the intaglio plate 111 by moving the stage 26 along the conveying direction 27.

The manufacturing apparatus 110 includes a liquid body supplying unit 130. The liquid body supplying unit 130 includes a dispenser 131. A liquid body F that contains the formation material of the acicular body 1 is discharged, such as to enter the recess 23, from the dispenser 131 onto the recess forming surface 22 of the intaglio plate 111 that is conveyed by the conveying unit 125. The liquid body supplying unit 130 may include an ink jet, a die coater, or the like instead of the dispenser 131.

The manufacturing apparatus 110 includes a film covering unit 35. The film covering unit 35 supplies a film 36 that covers the recess 23 to the recess forming surface 22 onto which the liquid body F has been discharged. The film covering unit 35 includes a feeding device 37, an intermediate roller 38, and a feeding roller.

The feeding device 37 supplies the film 36 from a whole film that is the shape of a roll to the intermediate roller 38. The intermediate roller 38 is rotatably supported such that the rotation center is a rotating shaft 39 that extends in a direction perpendicular to the conveying direction 27 of the stage 26. The intermediate roller 38 supplies the film 36 that has been unwound from the feeding device 37 to a pressing roller 41 of a pressing unit 40 that is a feeding roller. The pressing roller 41 of the pressing unit 40 supplies the film 36 supplied from the intermediate roller 38 to the recess forming surface 22, and covers the recess 23 on the recess forming surface 22 with the film 36.

The film 36 is a film material that can be permeated with the liquid body F discharged onto the recess forming surface 22. As the film 36, a non-woven fabric, a porous film, an air-permeable film such as fibers, or a water-soluble material film can be used. The film covering unit 35 may include a feeding apparatus that is capable of supplying sheets of film, instead of the feeding device 37.

The manufacturing apparatus 110 includes the pressing unit 40 that configures the de-airing unit. The pressing unit 40 presses the film 36 covering the recessing portion 23 against the recess forming surface 22. The pressing unit 40 includes the pressing roller 41 that presses the film 36 against the recess forming surface 22 and a platen roller 42 that supports the undersurface of the stage 26. The pressing unit 40 presses the film 36 against the recess forming surface 22 by sandwiching the stage 26, the intaglio plate 11, and the film 36. The rollers 41 and 42 are each rotatably supported such that the respective rotation centers are rotating shafts 43 and 44 that extend in a direction perpendicular to the conveying direction 27 of the stage 26. The pressing roller 41 also functions as a feeding roller that configures the film covering unit, as described above.

The pressing unit 40 is configured such that the pressing roller 41 and the platen roller 42 can be displaced in the up/down direction that is the normal direction of the setting surface 26a, as indicated by the arrows in the drawing. In the pressing unit 40, the pressing force of the film 36 against the recess forming surface 22 is changed depending on the position of the pressing roller 41 in relation to the platen roller 42 and the rubber hardness of the pressing roller 41. The pressing unit 40 is configured such as to be capable of vibrating the intaglio plate 111 by continuous displacement of the rollers 41 and 42 in the up/down direction. The direction in which each roller 41 and 42 is vibrated is merely required to a direction perpendicular to the conveying direction 27, and is not limited to the up/down direction. The feeding roller of the film covering unit and the pressing roller of the pressing unit may be separate rollers.

The manufacturing apparatus 110 includes an auxiliary film covering unit 45. The auxiliary film covering unit 45 supplies an auxiliary film 46 such as to cover the film 36 that is pressed against the recess forming surface 22 by the pressing unit 40. The auxiliary film covering unit 45 includes a feeding device 47 and a feeding roller 48 for the auxiliary film 46.

The feeding device 47 supplies the auxiliary film 46 from a whole film that is in the shape of a roll to the feeding roller 48. The feeding roller 48 supplies the auxiliary film 46 that has been unwound from the feeding device 47 to the intaglio plate 111 and covers the film 36 with the auxiliary film 46.

The auxiliary film 46 is a film material that has a passage that enables moisture that is generated with the drying of the liquid body F to be discharged outside and minimizes warpage of the substrate 11 that accompanies drying. The auxiliary film 46 is preferably of a material that has higher mechanical strength than the liquid body F after solidification, and is easily peeled from the liquid body F after drying. The auxiliary film covering unit 45 may be omitted.

### (Electrical configuration of the manufacturing apparatus)

An electrical configuration of the manufacturing apparatus 110 will be described with reference to Fig. 35.

As shown in Fig. 35, the manufacturing apparatus 110 includes a control unit 50 that is mainly configured by a microcomputer. The microcomputer has a central processing unit (CPU), a non-volatile memory (read-only memory (ROM)), and a volatile memory (random access memory (RAM)). The control unit 50 performs various processes related to the manufacture of the acicular body 1 based on various control programs and various data stored in the ROM.

The control unit 50 is electrically connected to an operating unit 151 that is configured by various buttons, a touch panel, and the like. The operating unit 515 outputs, to the control unit 50, various conditions, such as the thickness of the intaglio plate 111, the spacing of recess patterns, and the amount of discharge of the liquid body F, for manufacturing the acicular body 1 in the manufacturing apparatus 110. The control unit 50 starts various processes by the operation of a start button provided in the operating unit 151.

The control unit 50 is electrically connected to a conveyance amount detector 152 that detects the conveyance amount of the stage 26. The control unit 50 acquires the conveyance amount of the stage 26 by performing various calculations based on the detection signals from the conveyance amount detector 152.

The control unit 50 is electrically connected to a tension detector 153 that detects the tension of the film 36 between the intermediate roller 38 and the pressing roller 41. The control unit 50 acquires the tension of the film 36 by performing various calculations based on the detection signals from the tension detector 153.

The control unit 50 is electrically connected to a tension detector 154 that detects the tension of the auxiliary film 46 between the feeding device 47 and the feeding roller 48. The control unit 50 acquires the tension of the auxiliary film 46 by performing various calculations based on the detection signals from the tension detector 154.

When the start button in the operating unit 151 is operated, the control unit 50 performs a positioning process to set the position of the intaglio plate 111 in relation to the dispenser 131 to a start position. The positioning process is, for example, performed using a positioning mark (not shown) provided on the intaglio plate 111, a light source that irradiates an illumination light on the positioning mark, and an optical sensor that detects the diffraction light emitted from the positioning mark. The control unit 50 irradiates the illumination light from the light source onto the positioning mark, and detects the diffraction light emitted from the positioning mark using the optical sensor. Then, the control unit 50 drives the conveying unit 125 based on the diffraction light detected by the optical sensor, and disposes the intaglio plate 111 at the start position.

When the positioning process is completed, the control unit 50 performs a pressing force setting process to set the pressing force of the film 36 against the recess forming surface 22. In the pressing force setting process, the control unit 50 drives the pressing unit 40 based on various conditions inputted via the operating unit 151 and displaces the pressing roller 41 to a positon based on the various conditions, or in other words, to a position at which the pressing force is that suitable for the various conditions.

When the pressing force setting process is completed, the control unit 50 performs a conveying process for conveying the intaglio plate 11 set on the stage 26 at a predetermined conveying speed. In the conveying process, the control unit 50 calculates the conveying speed based on the detection signals from the conveying amount detector 152 and drives the conveying unit 125 such that the conveying speed is kept at a predetermined speed.

When the conveying process is started, the control unit 50 performs a discharging process for discharging the liquid body F from the dispenser 131 onto the recess forming surface 22. In the discharging process, the control unit 50 calculates the conveying amount of the intaglio plate 111 from the start position based on the detection signals from the conveying amount detector 152, and discharges the liquid body F from the dispenser 131 when the recess pattern arrives directly below the dispenser 131.

When the conveying process is started, the control unit 50 performs a film covering process in which the film 36 is unwound from the feeding device 37 and the recess 23 is covered by the film 36. In the film covering process, the control unit 50 drives the film covering unit 35 based on the detection signals from the tension detector 153, and unwinds the film 36 from the feeding device 37 such that the tension between the intermediate roller 38 and the pressing roller 41 is held at a predetermined value.

When the conveying process is started, the control unit 50 performs a vibrating process to vibrate the intaglio plate 111. In the vibrating process, the control unit 50 drives the pressing unit 40 and displaces the pressing roller 41 and the platen roller 42 at the same timing and by the same amount in the up/down direction.

When the conveying process is started, the control unit 50 performs an auxiliary film covering process in which the auxiliary film 46 is unwound from the feeding device 47 and the film 36 is covered by the auxiliary film 46. In the auxiliary film covering process, the control unit 50 drives the auxiliary film covering unit 45 based on the detection signals from the tension detector 154, and unwinds the auxiliary film 46 from the feeding device 47 such that the tension between the feeding device 47 and the feeding roller 48 is held at a predetermined value.

### (Manufacturing method for the acicular body 1)

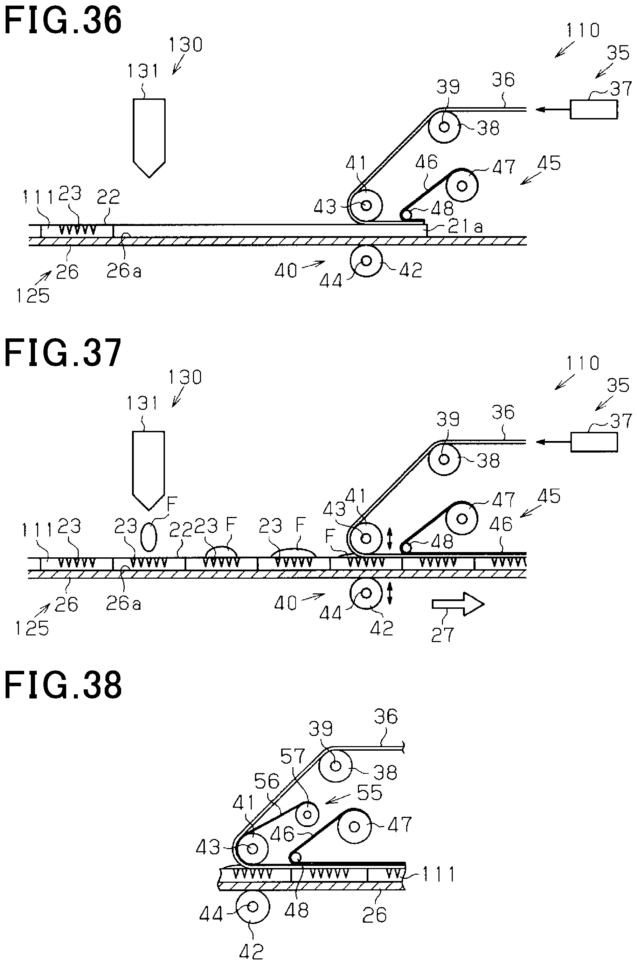

A manufacturing method for the acicular body 1 including the working mode of the manufacturing apparatus 110 will be described with reference to Fig. 36 and Fig. 37.

The manufacturing method for the acicular body 1 includes a liquid body discharging step, a film covering step, a film pressing step, an auxiliary film covering step, a drying step, and a removing step. Among these steps, the manufacturing apparatus 110 performs four steps that are the liquid body discharging step, the film covering step, the film pressing step, and the auxiliary film covering step.

As shown in Fig. 36, first, the intaglio plate 111 is placed on the setting surface 26a of the stage 26. At this time, an end portion 21a of the intaglio plate 111 passes through the space between the stage 26 and the pressing roller 41 and the space between the stage 26 and the feeding roller 48, and is disposed further downstream of the feeding roller 48 in the conveying direction 27 of the stage 26. In the end portion 21a of the intaglio plate 111, the film 36 is attached to the intaglio plate 111 and the auxiliary film 46 is attached to the film 36.

Next, when the various conditions are inputted to the control unit 50 through the operating unit 151 and the start button in the operating unit 151 is then operated, the control unit 50 performs the positioning process and an initial position setting process. Then, the control unit 50 starts the conveying process, the discharging process, the film covering process, the auxiliary film covering process, and the vibrating process.

As shown in Fig. 37, the liquid body F is discharged from the dispenser 131 onto the recess forming surface 22 of the intaglio plate 111 conveyed by the conveying unit 125, when the recess pattern arrives directly below the dispenser 131. As a result of vibration of the intaglio plate 111 by the pressing unit 40, the discharged liquid body F is prompted to wet and spread into the recess forming surface 22 during the conveying process of the intaglio plate 111.

Subsequently, the film 36 that covers the recess 23 is supplied to the recess forming surface 22 of the intaglio plate 111 by the pressing roller 41. The film 36 is presses against the recess forming surface 22 by the pressing roller 41. At this time, the pressing positon of the film 36 by the pressing roller 41 continuously changes along the conveying direction 27 in accompaniment with the conveyance of the intaglio plate 111.

As a result of the pressing position moving in this way, the state is such that the intaglio plate 111, the liquid body F, and the film 36 are handled by the pressing roller 41. Therefore, the flow of the liquid body F is promoted. As a result, air bubbles that are included in the liquid body F are discharged outside of the liquid body F through the space between the recess forming surface 22 and the film 36 that is not yet pressed or through the film 36. As a result, the liquid body F fills all the way to the intricate portions of the recess 23. Furthermore, because the flow of the liquid body F is further promoted by the vibration of the intaglio plate 111 by the pressing unit 40, air bubbles are more easily discharged from the liquid body F.

Next, the auxiliary film 46 that covers the film 36 is provided to the intaglio plate 111 by the feeding roller 48. Then, the intaglio plate 111 is conveyed by the conveying unit 125 such that the drying step, which is the next step, is performed. At the drying step, warpage of the substrate 11 caused by drying of the liquid body F is minimized by the auxiliary film 46.

Then, as a result of the liquid body F solidifying in the intaglio plate 111 that has completed the drying step, a molded article in which the film 36 is included in a portion of the substrate 11 is formed such as to be sandwiched between the intaglio plate 111 and the auxiliary film 46. At the removing step, the molded article is removed from the intaglio plate 111 and the auxiliary film 46, and the acicular body 1 is thereby obtained.

According to the above-described seventh embodiment, the advantageous effects listed below can be achieved.
(10) As a result of the film 36 being pressed against the recess forming surface 22, the accuracy of the shape of the acicular body 1 is improved.
(11) As a result of the conveyance of the intaglio plate 111 by the conveying unit 125 and the pressing of the film 36 by the pressing unit 40 being simultaneously performed, the cycle time of the acicular body 1 in the manufacturing apparatus 110 is shortened. As a result, productivity of the acicular body 1 is improved.
(12) As a result of the intaglio plate 111 being linearly conveyed along the conveying direction 27 by the conveying unit 125, the intaglio plate 111 and the pressing unit 40 move in relation to each other. Therefore, compared to when a plurality of directions are set as the directions in which the intaglio plate 11 and the pressing unit 40 move in relation to each other, the configuration of the apparatus can be simplified.
(13) The intaglio plate 111 moves in relation to the liquid body supplying unit 130, the film covering unit 35, and the pressing unit 40. Therefore, the moving unit that moves the intaglio plate 11 in relation to the liquid body supplying unit 130, the film covering unit 35, and the pressing unit 40 is merely required to be the conveying unit 125 that conveys the intaglio plate 111. As a result, the configuration of the moving unit can be simplified.
(14) As a result of the pressing of the film 36 being performed by the pressing roller 41, pressing of the film 36 and conveyance of the intaglio plate 111 can be smoothly performed.
(15) As a result of the feeding roller of the film covering unit 35 and the pressing roller 41 of the pressing unit 40 being shared, space-saving and reduction of constituent elements of the manufacturing apparatus 110 can be achieved.
(16) As a result of the vibration of the intaglio plate 11, air bubbles can be easily discharged from the liquid body F.
(17) As a result of the vibration of the intaglio plate 11, the liquid body F more easily wets and spreads over the recess forming surface 22.
(18) Because the film 36 can be permeated with the liquid body F, the degree of freedom regarding the discharge path of air bubbles is increased and the air bubbles become more easily discharged.
(19) The film 36 is a non-woven fabric, a porous film, or an air-permeable film, such as fibers. Therefore, the air bubbles can also be discharged outside of the liquid body F through the film 36.
(20) As a result of the auxiliary film 46 being supplied to the intaglio plate 111, deformation of the acicular body 1 accompanying the drying of the liquid body F is minimized.
(21) In the manufacturing apparatus 110, supplying of the liquid body F by the liquid body supplying unit 130, supplying of the film 36 by the film covering unit 35, and pressing of the film 36 by the pressing unit 40 are performed in this order on the intaglio plate 111 conveyed by the conveying unit 125. Therefore, in the manufacturing apparatus 110, for example, supplying of the liquid body F and supplying of the film 36 can be simultaneously performed on differing recess patterns. As a result, the cycle time in the manufacturing apparatus 110 can be shortened.
(22) As a result of wafer-paper film being used as the film 36, the film 36 is not observed in the acicular body 1 after molding. That is, the outer appearance of the acicular body 1 is not diminished by the film 36.

The above-described seventh embodiment can be carried out with the following modifications, as appropriate.
- The film 36 is not limited to a non-woven fabric, a porous film, an air-permeable film, such as fibers, or a water-soluble material film. The film 36 may be a film that seals the recess 23 after pressing by the pressing roller 41. In a configuration such as this as well, air bubbles are discharged from the liquid body F through the space between the recess forming surface 22 and the film 36 that is not yet pressed, as a result of the film 36 being pressed against the recess forming surface 22.
- The pressing unit 40 is only required to press the film 36 against the recess forming surface 22 and does not necessarily have to vibrate the intaglio plate 111 during conveyance. When the intaglio plate 111 is vibrated, the manufacturing apparatus 110 may include a vibrating unit as a mechanism separate from the pressing unit 40.
- The pressing unit 40 is merely required to be a mechanism that presses the film 36 against the recess forming surface 22. Therefore, for example, the pressing unit 40 may have a fixed member, instead of the rotatable pressing roller 14, as a pressing member that presses the film 36 against the recess forming surface 22. The fixed member includes, as a pressing surface, a curved surface that protrudes towards the recess forming surface 22, which is fixed to a bracket or the like.
- The conveying unit is not limited to the conveying unit 125 that conveys the stage 26, on which the intaglio plate 111 is set, along the conveying direction 27. For example, the conveying unit may be configured such that either one of the pressing roller 41 and the platen roller 42 is a driving roller and the other is a driven roller, and the intaglio plate 11 is conveyed by the rotation of the driving roller.
- The moving unit is not limited to the conveying unit 125 that conveys the intaglio plate 111 along the conveying direction 27. The moving portion is merely required to be a mechanism that relatively moves the intaglio plate 111 and the pressing unit 40. Therefore, for example, the moving unit may be a mechanism that moves the liquid body supplying unit 130, the film covering unit 35, and the pressing unit 40 as a single unit in relation to the intaglio plate 111. For example, the moving unit may be a mechanism that moves the intaglio plate 111 along a plurality of directions in relation to the liquid body supplying unit 130, the film covering unit 35, and the pressing unit 40.
- The manufacturing apparatus 110 is merely required to include a state where the film 36 is pressed while the intaglio plate 111 and the pressing unit 40 are moved in relation to each other, as a pressing state where the film 36 is pressed against the recess forming surface 22. Therefore, the pressing state may include a state where the relative movement between the intaglio plate 111 and the pressing unit 40 is stopped while the film 36 remains pressed against the recess forming surface 22.
- The manufacturing apparatus 110 may be provided with a drying unit that is a mechanism for drying the liquid body F, downstream from the auxiliary film covering unit 45. The manufacturing apparatus 110 may also be provided with an auxiliary film peeling unit that is a mechanism for peeling the auxiliary film, downstream from the drying unit.
- The liquid body supplying unit 130 may supply the liquid body F into the recess 23 over a plurality of times. As a result of a configuration such as this, the degree of freedom regarding the size of the substrate 11 is improved.
- The auxiliary film 46 is a film that minimizes warpage of the substrate 11 that accompanies drying of the liquid body F. Therefore, the auxiliary film 46 may be disposed such that a predetermined space is created in relation to the film 36.
- As shown in Fig. 38, the manufacturing apparatus 110 may further include a cover film covering unit 55. The cover film covering unit 55 supplies a cover film 56 that covers the film 36 to the intaglio plate 111. The cover film covering unit 55 supplies the cover film 56 to the pressing roller 41 from a feeding device 57 that includes a whole film in the shape of a roll, thereby inserting the cover film 56 between the film 36 and the pressing roller 41. The pressing roller 41 supplies a film composed of a multilayer structure of the film 36 and the cover film 56 to the intaglio plate 111, and presses the film composed of a multilayer structure against the recess forming surface 22.

In a configuration such as this, the control unit 50 is connected to a tension detector that detects the tension of the cover film 56 between the feeding device 57 and the pressing roller 41. The control unit 50 acquires the tension of the cover film 56 by performing various calculations based on the detection signals from the tension detector. The control unit 50 drives the feeding device 57 based on the tension of the cover film 56 between the feeding device 57 and the pressing roller 41.
- The setting atmosphere of the manufacturing apparatus 110 may be any of a normal pressure atmosphere, a reduced pressure atmosphere, and a pressurized atmosphere.

### [Eighth embodiment]

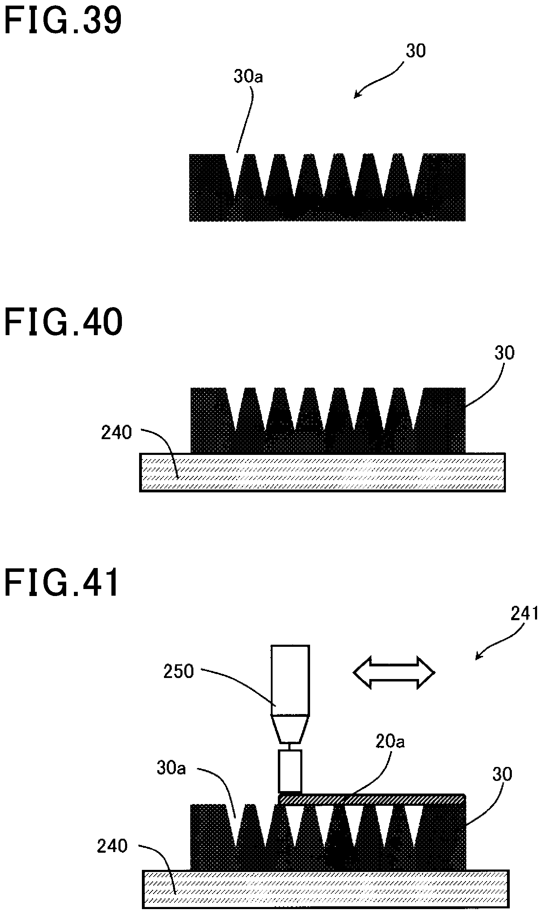

A method for manufacturing an acicular body by a sheet method will be described with reference to Fig. 39 to Fig. 45.

### <Preparing step>

First, as shown in Fig. 39, an intaglio plate 30 having a recess 30a that is shaped such as to correspond to the needle is prepared.

Next, as shown in Fig. 40, the intaglio plate 30 is mounted on a table 240 that holds the intaglio plate 30 level. As a result of a level table such as a surface plate, for example, being used as the table 240, a needle forming solution 20a can be supplied at a more even film thickness at a supplying step, which is a subsequent step.

### <Coating step>

Next, the intaglio plate 30 is coated with the needle forming solution 20a (coating step). The coating method can be selected as appropriate from publically known methods, taking into consideration the shape and dimensions of the intaglio plate 30 and the like. For example, a coating apparatus 241 to which a smooth nozzle-attached dispenser 250 is attached may be used on the intaglio plate 30 that is set on the table 240 as shown in Fig. 41. As a result, the needle forming solution 20a can be supplied at the desired film thickness and area. Supplying of the needle forming solution 20a to the intaglio plate 30 may be performed under normal pressure. However, to more favorably fill the recess 30a with the needle forming solution 20a, supplying may be performed under reduced pressure or in a vacuum. The amount of needle forming solution 20a is preferably that which covers all recesses 30a.

### <Film transferring step>

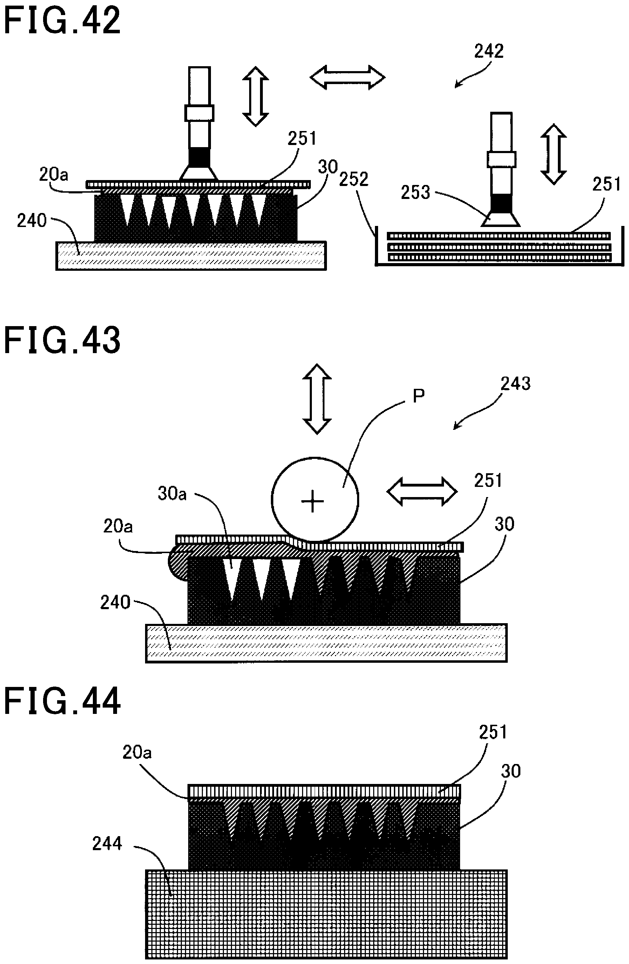

Next, a film 251 is transferred to the intaglio plate 30 to which the needle forming solution 20a has been supplied (film transferring step). As shown in Fig. 42, the film 251 is housed within a film housing body 252. The film 251 can be efficiently supplied by a film conveying mechanism that is capable of moving the film 251 to the intaglio plate 30 supplied with the needle forming solution 20a by a transferring apparatus 242. The transferring apparatus 242 can transfer the film without damaging the film by using a suction pad 253, for example.

As the formation material of the film 251, the needle formation material used in the needle formation liquid, described above, in the form of a film can be used. Specifically, a water-soluble material film composed of a water-soluble polymer or a polysaccharide can be used. As the formation material of the film 251, a non-woven fabric, a porous film, or an air-permeable film such as fibers can also be used. The film 251 can be composed of the same material as the needle forming solution 20a. The film 251 does not necessarily have to be composed of the same material as the needle forming solution 20a. When the materials differ, a two-layer acicular body can also be manufactured.

### <Compressing step>

Next, a press member P is placed in contact with the film 251, and the film 251 is compressed (compressing step). A circular cylindrical member can be used as the press member P. As shown in Fig. 43, as a result of a circular cylindrical roll being used, a compressing apparatus 243 that is capable of descending onto the intaglio plate 30 supplied with the needle forming solution 20a and the film 251 and pressing while horizontally moving from one end portion of the intaglio plate 30 to the other end portion can be used. As a result, productivity can be improved. An elastic body, such as that of rubber or resin, is used as the material for the press member P. Regarding hardness, the Shore hardness preferably ranges from Shore A (JIS K6253 (1993) Type A durometer) 50 to 90, both inclusive. When Shore A is less than 50, the press member P may adhere to the film 251 during compression and become inseparable. Meanwhile, when Shore A exceeds 90, when a water-soluble polymer or a polysaccharide is used as the film 251, the film 251 may be dissolved in the needle forming solution 20a.

Compression is preferably performed such that the load during descending of the press member P ranges from 14 kgf to 300 kgf. When the load during descending of the press member P is less than 14 kgf, filling insufficiency may occur. Meanwhile, when the load during descending of the press member P exceeds 300 kgf, the intaglio plate 30 may become deformed.

In the compressing apparatus 243, described above, filling insufficiency may occur when the horizontal movement speed is low, as well as when high. Therefore, the rotation movement speed in the horizontal direction preferably ranges from 2 mm/sec to 100 mm/sec. When the relative rotation movement speed in the horizontal direction is less than 2 mm/sec, the manufacturing time increases, and manufacturing cost may increase. When the relative rotation movement speed in the horizontal direction is less than 2 mm/sec, the manufacturing time increases, and manufacturing cost may increase. When the relative rotation movement speed in the horizontal direction exceeds 100 mm/sec, there is a probability that filling is not performed up to the pointed bottom end of the recess.

As a result of the compressing step, the needle forming solution 20a is compressed and moves into each recess 30a.

### <Drying step>

Next, the liquid component within the needle forming solution 20a is removed, and the needle forming solution 20 is solidified, thereby forming the needle 20 (drying step).

As shown in Fig. 44, the drying step can be performed by holding and drying at normal temperature. However, heat-drying of the needle forming solution 20a by heating the intaglio plate 30 or the like is preferable become required time can be shortened.

When the needle forming solution 20a is heated, air bubbles may form. However, because the gas inside the air bubbles pass through the film 251 that serves as a support substrate, the air bubbles are favorably removed from within the recess 30a. However, it is most preferable that air bubbles themselves are not formed. Therefore, the heating temperature is preferably to a degree than the needle forming solution 20a does not boil, and for example, ranges from 40°C to 90°C. The heating method is not particularly limited. For example, a hot plate on which the intaglio plate 30 is placed can be used. After formation of the needle 20, the acicular body 1 that includes the support substrate 10 and the needle 20 is completed.

The acicular body 1 may be punched into the desired size and shape depending on the intended use and the like, after being peeled from the intaglio plate 30. Punching may be performing using a punching blade, such as a Thomson blade.

As a result of an adhesive being attached to the periphery of the needle, an acicular body that can be attached to the skin or the like is formed. An adhesive composed of a material that is suitable for attachment to the skin is preferably used. An adhesive capable of withstanding a sterilizing step is more preferable.

### <Peeling step>

Finally, as a result of the acicular body 1 being peeled from the intaglio plate, the acicular body 1 is manufactured.

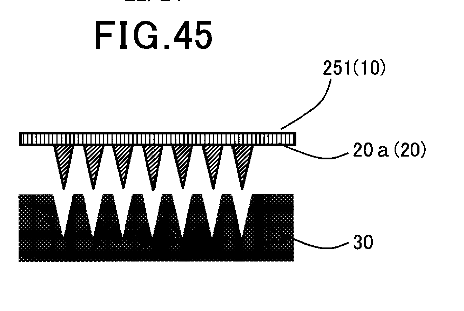

As shown in Fig. 45, when the acicular body 1 is removed from the intaglio plate 30, the acicular body 1 may be mechanically peeled from the intaglio plate 30 or the intaglio plate 30 may be chemically dissolved.

As described above, as a result of the manufacturing method for an acicular body according to the present embodiment, at the filling step, the needle forming solution 20a is compressed in a state where the film 251 is disposed on the needle forming solution 20a. Therefore, the recess 30a is favorably filled with the needle forming solution 20a. Furthermore, at the solidifying step, the vaporized liquid component of the needle forming solution 20a can be transmitted through the film 251, serving as the support substrate 10, and transpired. Therefore, the needle can be formed by the needle forming solution 20a being favorably dried and solidified, while the film 251 remains in place.

As a result, an acicular body can be manufactured at low cost and under atmospheric pressure, while minimizing mingling of air bubbles.

A manufacturing apparatus for an acicular body
will be described.

The manufacturing apparatus is a manufacturing apparatus that is capable of carrying out the above-described manufacturing method for an acicular body of the present invention. The manufacturing apparatus includes at least a table, a coating apparatus, a film transferring apparatus, and a compressing apparatus. The table holds level an intaglio plate that has a recess of a shape corresponding to the needle. The coating apparatus fills the intaglio plate having the recess of a shape corresponding to the needle with a needle formation liquid in which an acicular body material is dissolved or dispersed in a solvent. The film transferring apparatus disposes a film on the needle formation liquid on the intaglio plate. The compressing apparatus includes a compressing body that has a circular cylindrical elastic body capable of applying load on the intaglio plate in the up/down direction.

At this time, the table that holds the intaglio plate level is preferably capable of moving and conveying between the coating apparatus, the film transferring apparatus, and the compressing apparatus.

The manufacturing method for an acicular body of the present invention will be further described using examples and comparative examples. However, the technical scope of the present invention is not limited in any way by the examples and comparative examples.

### [Examples according to the first and second embodiments]

### [Example 1]

### <Fabrication of an intaglio plate>

An acicular-body original plate was formed using precision-machine machining. In the acicular-body original plate, a total of 36 square cones (height: 150 µm; base: 60 µm × 60 µm) are arrayed on a silicon substrate, at 1 mm intervals, in a grid of six rows and six columns. A nickel film having a thickness of 500 µm was formed by a plating method on the acicular-body original plate. The silicon substrate was removed by wet etching with a potassium hydroxide aqueous solution heated to 90°C and having a weight percent concentration of 30%. An intaglio plate composed of nickel and having 36 recesses was fabricated.

### <Preparation of a needle forming solution>

Chitosan succinamide was dissolved in water, and a chitosan succinamide aqueous solution having a weight percent concentration of 10% was prepared.

### <Fabrication of an acicular body>

The above-described needle forming solution was supplied onto the intaglio plate by a dropper such as to cover all recesses. A porous film (polyethylene material, thickness of 500 µm) was disposed on the needle forming solution.

The experimenter grasped a rubber rod in the hands and performed compression with the rubber rod from above the porous film. After compression was stopped, the intaglio plate was placed on a hot plate and heated at 90°C for 10 minutes. The needles were dried and solidified, and the needles were thereby formed.

After needle formation, the porous film surrounding the needles was punched into a circle. An adhesive was attached in the area surrounding the needles on the surface on the needle side. Finally, the intaglio plate was removed from the needles, and an acicular body of example 1 was obtained.

### [Example 2]

A film composed of chitosan succinamide and having a thickness of 200 µm was used as the film. Otherwise, an acicular body of example 2 was obtained by a process similar to that of example 1.

### [Example 3]

An HPC aqueous solution having a weight percent concentration of 10% was used as the needle forming solution. A film composed of HPC and having a thickness of 100 µm was used as the film. Otherwise, an acicular body of example 3 was obtained by a process similar to that of example 1. The heating temperature was 40°C and the heating time was a period of 20 minutes.

### [Example 4]

Rhodamine B was mixed into the needle forming solution at a weight percent of 0.2%. Otherwise, an acicular body of example 4 was obtained by a process similar to that of example 3.

Examples 5 to 9 are examples in which other water-soluble polymers were used as the material.

### [Example 5]

A pullulan aqueous solution having a weight percent concentration of 10% was used as the needle forming solution. A film composed of pullulan and having a thickness of 200 µm was used as the film. Otherwise, an acicular body of example 5 was obtained by a process similar to that of example 1.

### [Example 6]

An alginate aqueous solution having a weight percent concentration of 10% was used as the needle forming solution. A film composed of alginate and having a thickness of 200 µm was used as the film. Otherwise, an acicular body of example 6 was obtained by a process similar to that of example 1.

### [Example 7]

A pectin aqueous solution having a weight percent concentration of 10% was used as the needle forming solution. A film composed of pectin and having a thickness of 200 µm was used as the film. Otherwise, an acicular body of example 7 was obtained by a process similar to that of example 1.

### [Example 8]

A CMC aqueous solution having a weight percent concentration of 10% was used as the needle forming solution. A film composed of CMC and having a thickness of 200 µm was used as the film. Otherwise, an acicular body of example 8 was obtained by a process similar to that of example 1.

### [Example 9]

An HPMC aqueous solution having a weight percent concentration of 10% was used as the needle forming solution. A film composed of HPMC and having a thickness of 200 µm was used as the film. Otherwise, an acicular body of example 9 was obtained by a process similar to that of example 1.

Examples 10 to 12 are examples used to examine the effects of various parameters, such as the concentration of the needle forming solution, the film thickness, and drying conditions.

### [Example 10]

An HPC aqueous solution having a weight percent concentration of 5% was used as the needle forming solution. A film composed of HPC and having a thickness of 100 µm was used as the film. Otherwise, an acicular body of example 10 was obtained by a process similar to that of example 3. The heating temperature was 40°C and the heating time was a period of 10 minutes.

### [Example 11]

An HPC aqueous solution having a weight percent concentration of 50% was used as the needle forming solution. A film composed of HPC and having a thickness of 100 µm was used as the film. Otherwise, an acicular body of example 10 was obtained by a process similar to that of example 3. The heating temperature was 40°C and the heating time was a period of 5 minutes.

### [Example 12]

An HPC aqueous solution having a weight percent concentration of 30% was used as the needle forming solution. Otherwise, an acicular body of example 12 was obtained by a process similar to that of example 10.

Examples 13 to 16 are examples of a so-called two-layer acicular body in which differing water-soluble polymers were used in the needle forming solution and the film.

### [Example 13]

A film composed of HPC and having a thickness of 100 µm was used as the film. Otherwise, an acicular body of example 13 was obtained by a process similar to that of example 1.

### [Example 14]

A film composed of HPC and having a thickness of 200 µm was used as the film. Otherwise, an acicular body of example 14 was obtained by a process similar to that of example 5.

### [Example 15]

A film composed of pullulan and having a thickness of 200 µm was used as the film. Otherwise, an acicular body of example 15 was obtained by a process similar to that of example 8.

### [Example 16]

A methylcellulose aqueous solution having a weight percent concentration of 10% was used as the needle forming solution. Otherwise, an acicular body of example 16 was obtained by a process similar to that of example 2. The heating temperature was 40°C and the heating time was a period of 20 minutes.

Examples 17 to 19 are examples corresponding to the manufacturing process of the modification, described above.

### [Example 17]

A film composed of HPC and having a thickness of 100 µm was used as the film. One surface of the film was sprayed and wetted with water. The sprayed surface was then adhered to the intaglio plate before the water had dried, and compression was performed in a manner similar to that in example 1. Plasma treatment was performed in advance on the surface of the intaglio plate on the side provided with the recesses. After compression, heating was performed at 40°C for a period of 20 minutes, and an acicular body of example 17 was obtained.

### [Example 18]

A film composed of HPC and having a thickness of 100 µm was used as the film. The surface of the intaglio plate on the side provided with the recesses was sprayed and wetted with water. The film was adhered to the intaglio plate before the water had dried, and compression was performed in a manner similar to that in example 1. After compression, heating was performed at 40°C for a period of 20 minutes, and an acicular body of example 18 was obtained.

### [Example 19]

An HPC aqueous solution having a weight percent concentration of 10% was used as the needle forming solution. A porous film composed of polyethylene and having a thickness of 500 µm was used as the film. One surface of the film was coated with the needle forming solution. The coated surface was adhered to the intaglio plate before the needle forming solution had dried, and compression was performed in a manner similar to that in example 1. Plasma treatment was performed in advance on the surface of the intaglio plate on the side provided with the recesses. After compression, heating was performed at 40°C for a period of 20 minutes, and an acicular body of example 19 was obtained.

### [Comparative example 1]

A film was not disposed and compression was not performed. Otherwise, an acicular body of comparative example 1 was obtained by a process substantially similar to that of example 1. The heating temperature was 90°C and the heating time was a period of 30 minutes.

### [Comparative example 2]

A film was not disposed and compression was not performed. Otherwise, an acicular body of comparative example 2 was obtained by a process substantially similar to that of example 3. The heating temperature was 40°C and the heating time was a period of 100 minutes. When compression is not performed, the supplied amount of the needle forming solution is greater than that in an example in which compression is performed. Therefore, the heating time is increased in accompaniment thereto.

### [Comparative example 3]

A PET film that is not porous and that has a film thickness of 100 µm was used as the film. Otherwise, an acicular body of comparative example 3 was obtained by a process similar to that of example 1.

The needles of the examples and the comparative examples were observed under a microscope, and the formation rate of the needles was calculated. The formation rate (%) was calculated by an expression: favorably formed needles / 36 × 100. A needle of which the vertex of the square cone can be confirmed and air bubbles are not found, based on microscopic observation, were determined to be "favorably formed".

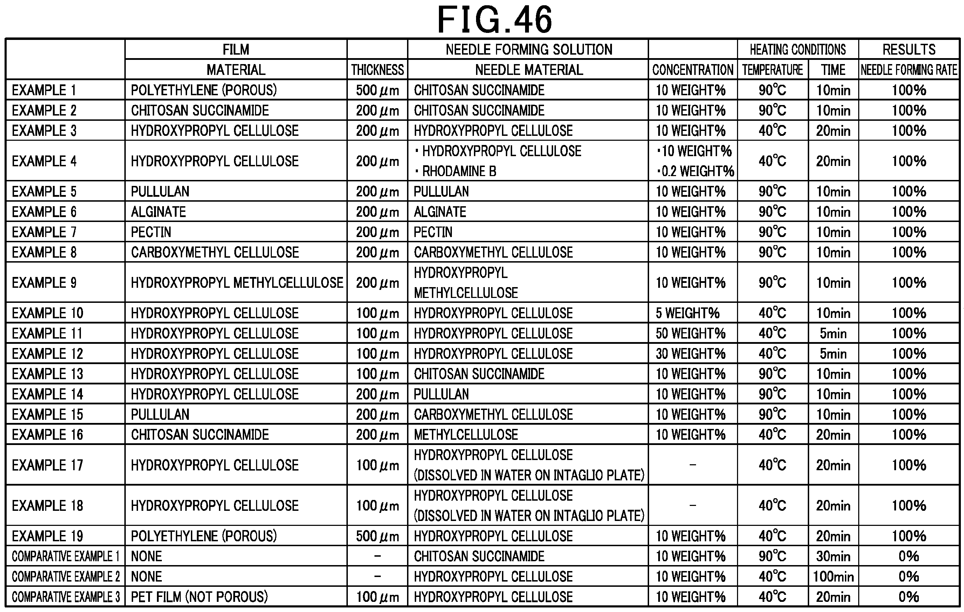

The results are shown in Fig. 46.

As shown in Fig. 46, the formation rate of the needles was 100% for all examples, but 0% for all comparative examples. In the comparative examples, needles having irregular shapes caused by air bubbles were observed.

When the acicular body of example 4 was visually observed from both sides in the thickness direction, certain surfaces of the acicular body were colored a light red, derived from rhodamine B. However, the back surface on the main body side retained the light white color of the HPC film. Therefore, it was thought that, in example 4, solute movement from the porous film was low, and an acicular body having a substantially two-layered structure composed of a layer containing rhodamine B and a layer not containing rhodamine B was fabricated.

As examples 10 to 12 indicate, as a result of the concentration of the needle material in the needle forming solution being high and the water-soluble material film serving as the support substrate being thin, the time required for drying and solidification could be shortened.

As examples 13 to 16 indicate, a two-layer acicular body could also be easily manufactured by the manufacturing method of the present invention.

As examples 17 to 19 indicate, it was confirmed that, in the manufacturing method the present invention, an acicular body could be favorably manufactured even when the needle forming solution was not disposed inside the recesses.

### [Examples according the third embodiment]

### [Example 20]

### <Fabrication of an intaglio plate>

An acicular-body original plate was formed using precision-machine machining. In the acicular-body original plate, a total of 36 circular cones (height: 150 µm; base diameter: 60 µm) are arrayed on a silicon substrate, at 1 mm intervals, in a grid of six rows and six columns. A nickel film having a thickness of 500 µm was formed by a plating method on the acicular-body original plate. The silicon substrate was removed by wet etching with a potassium hydroxide aqueous solution heated to 90°C and having a weight percent concentration of 30%. An intaglio plate composed of nickel and having 36 recesses was fabricated.

### <Preparation of a needle-side layer forming liquid>

Chitosan succinamide was dissolved in water, and a chitosan succinamide aqueous solution having a weight percent concentration of 10% was prepared. Evans blue was added to the prepared liquid to a concentration of 1%.

### <Fabrication of a film serving as a support substrate-side layer>

Chitosan succinamide was dissolved in water, and a chitosan succinamide aqueous solution having a weight percent concentration of 10% was prepared. Rhodamine B was added to the prepared liquid to a concentration of 1%. The liquid was dried and solidified by a casting method, and a chitosan succinamide/rhodamine B film was fabricated.

### <Fabrication of an acicular body>

The above-described needle-side layer forming liquid was supplied onto the intaglio plate by a dropper such as to cover all recesses. The above-described film was then disposed on the needle-side layer forming liquid.

The experimenter grasped a rubber rod in the hands and performed compression with the rubber rod from above the acicular body material film. After compression was stopped, the intaglio plate was placed on a hot plate and heated at 90°C for 10 minutes. The needles were dried and solidified, and the acicular body was thereby formed.

After acicular body formation, the film surrounding the needles was punched into a circle. An adhesive was attached in the area surrounding the needles on the surface on the needle side. Finally, the intaglio plate was removed from the needles, and an acicular body of example 20 was obtained.

### [Example 21]

The acicular body material film was fabricated from an aqueous solution of 10% hydroxypropyl cellulose / 2% ascorbic acid. Otherwise, an acicular body of example 21 was obtained by a process similar to that of example 20.

The needles of the examples were observed under a microscope, and the formation rate of the needles was calculated. The formation rate (%) was calculated by an expression: favorably formed needles / 36 × 100. A needle of which the vertex of the circular cone can be confirmed and air bubbles are not found, based on microscopic observation, were determined to be "favorably formed". As a result of microscopic observation, the formation rate of the needles was 100% for both example 20 and example 21.

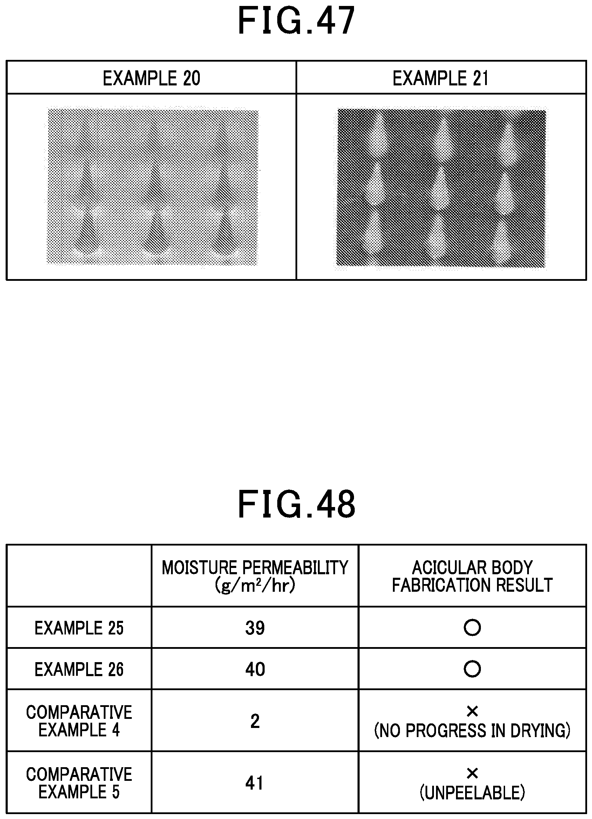

As a result of the acicular bodies of example 20 and example 21 being observed under a microscope, as shown in Fig. 47, it was found that coloring caused by contained substances differ between the needle and the substrate portion. The appearance of an acicular body having a two-layer structure could be confirmed. Further, as a result of the acicular bodies of example 20 and example 21 being observed under a microscope, no air bubbles or the like could be confirmed.

### [Example 22]

### <Fabrication of an intaglio plate>

An intaglio plate was fabricated in a manner similar to that of example 20.

### <Preparation of a needle-side layer forming liquid>

Chitosan succinamide was dissolved in water, and a chitosan succinamide aqueous solution having a weight percent concentration of 2% was prepared. Evans blue was added to the prepared liquid to a concentration of 1%.

### <Fabrication of a film serving as a support substrate-side layer>

Chitosan succinamide was dissolved in water, and a chitosan succinamide aqueous solution having a weight percent concentration of 10% was prepared. Rhodamine B was added to the prepared liquid to a concentration of 1%. The liquid was dried and solidified by a casting method, and a chitosan succinamide/rhodamine B film was fabricated.

### <Fabrication of an acicular body>

The above-described needle-side layer forming liquid was supplied onto the intaglio plate by a dropper such as to cover all recesses. The above-described film was then disposed on the needle-side layer forming liquid.

The intaglio plate was placed on a hot plate and heated at 90°C for 30 minutes. The needles were dried and solidified, and a needle-side layer was thereby formed.

Next, a drop of water was supplied to the needle-side layer by a dropper, and the above-described film was disposed on the needle-side layer. The experimenter grasped a rubber rod in the hands and performed compression with the rubber rod from above the acicular body material film.

Subsequently, the acicular body material film surrounding the needles was punched into a circle. An adhesive was attached in the area surrounding the needles on the surface on the needle side. Finally, the intaglio plate was removed from the needles, and an acicular body of example 22 was obtained.

As a result of the acicular body of example 22 being observed under a microscope, the formation rate of the needles was 100%. Further, it was found that coloring caused by contained substances differ between the needle-side layer and the substrate-side layer. The appearance of an acicular body having a two-layer structure could be confirmed. As a result of the acicular body of example 22 being observed under a microscope, no air bubbles or the like could be confirmed.

### [Example 23]

### <Fabrication of an intaglio plate>

An intaglio plate was fabricated in a manner similar to that of example 20.

### <Preparation of a needle-side layer forming liquid>

Chitosan succinamide was dissolved in water, and a chitosan succinamide aqueous solution having a weight percent concentration of 10% was prepared. Evans blue was added to the prepared liquid to a concentration of 1%.

### <Procurement of a film serving as a support substrate-side layer>

A nylon non-woven fabric was prepared.

### <Fabrication of an acicular body>

The above-described needle-side layer forming liquid was supplied onto the intaglio plate by a dropper such as to cover all recesses. The above-described film was then disposed on the needle-side layer forming liquid.

The experimenter grasped a rubber rod in the hands and performed compression with the rubber rod from above the acicular body material film. After compression was stopped, the intaglio plate was placed on a hot plate and heated at 90°C for 10 minutes. The needles were dried and solidified.

After solidification, a rhodamine 1% solution was sprayed from the film front surface onto the overall film, and thereby applied.

After acicular body formation, the film surrounding the needles was punched into a circle. An adhesive was attached in the area surrounding the needles on the surface on the needle side. Finally, the intaglio plate was removed from the needles, and an acicular body of example 23 was obtained.

The needles of the examples were observed under a microscope, and the formation rate of the needles was calculated. The formation rate (%) was calculated by an expression: favorably formed needles / 36 × 100. A needle of which the vertex of the circular cone can be confirmed and air bubbles are not found, based on microscopic observation, were determined to be "favorably formed".

As a result of the acicular body of example 23 being observed under a microscope, the formation rate of the needles was 100% for each. It was found that coloring caused by contained substances differ between the needle and the substrate portion. The appearance of an acicular body having a two-layer structure could be confirmed. As a result of the acicular body of example 23 being observed under a microscope, no air bubbles or the like could be confirmed.

### [Example 24]

### <Fabrication of an intaglio plate>

An intaglio plate was fabricated in a manner similar to that of example 20.

### <Preparation of a needle-side layer forming liquid>

Chitosan succinamide was dissolved in water, and a chitosan succinamide aqueous solution having a weight percent concentration of 2% was prepared. Evans Blue was added to the prepared liquid to a concentration of 1%.

### <Procurement of a film serving as a support substrate-side layer>

A nylon non-woven fabric was prepared.

### <Fabrication of an acicular body>

The above-described needle-side layer forming liquid was supplied onto the intaglio plate by a dropper such as to cover all recesses. The above-described film was then disposed on the needle-side layer forming liquid.

The intaglio plate was placed on a hot plate and heated at 90°C for 30 minutes. The needles were dried and solidified, and a needle-side layer was thereby formed.

Next, a drop of water was supplied to the needle-side layer by a dropper, and the above-described film was disposed on the needle-side layer. The experimenter grasped a rubber rod in the hands and performed compression with the rubber rod from above the acicular body material film.

After solidification, a rhodamine 1% solution was sprayed from the film front surface onto the overall film, and thereby applied.

After acicular body formation, the film surrounding the needles was punched into a circle. An adhesive was attached in the area surrounding the needles on the surface on the needle side. Finally, the intaglio plate was removed from the needles, and an acicular body of example 24 was obtained.

Subsequently, the acicular body material film surrounding the needles was punched into a circle. An adhesive was attached in the area surrounding the needles on the surface on the needle side. Finally, the intaglio plate was removed from the needles, and an acicular body of example 22 was obtained.

As a result of the acicular body of example 24 being observed under a microscope, the formation rate of the needles was 100% for each. Further, it was found that coloring caused by contained substances differ between the needle-side layer and the substrate-side layer. The appearance of an acicular body having a two-layer structure could be confirmed. As a result of the acicular body of example 24 being observed under a microscope, no air bubbles or the like could be confirmed.

### [Examples according to the fourth embodiment]

### <Fabrication of an acicular body>

### [Example 25]

An acicular body was fabricated by the following method.
(1) First, an acicular-body original plate was formed using precision-machine machining. In the acicular-body original plate, a total of 36 square cones (base: 38 µm × 38 µm; height: 120 µm) are arrayed on a silicon substrate, at 1 mm intervals, in a grid of six rows and six columns.
(2) Next, a mold-release treatment was performed on the acicular-body original plate formed from the silicon substrate, using NOVEC (registered trademark) EGC-1720, a fluorine-based coating agent by Ryoko Chemicals Co., Ltd.
(3) KU-5550, a urethane for molding manufactured by Hitachi Chemical Co., Ltd., was supplied to the original plate that had been subjected to the mold-release treatment at (2). Curing was performed at 150°C, and an intaglio plate was thereby fabricated.
(4) A 20 weight% aqueous solution of hydroxypropyl cellulose (Tokyo Chemical Industry Co., Ltd.; 6 to 10 mPa·s; 2% in water at 20°C) was dropped onto the intaglio plate fabricated at (3), using a dropper, to an extent that an acicular pattern was covered.
(5) Pressing at a load of 90 N in pressing mode was performed using TENSILON universal material testing machine (A&D Company, Ltd.) to which a cylindrical rod composed of nitrile rubber and having a diameter of 16 mm is attached, over a silicone-coated fibrous sheet (Lion Corporation; REED (registered trademark) Healthy Cooking Sheet).
(6) The intaglio plate at (5) was dried by being placed on a hot plate set to 40°C for 8 hours, and moisture was evaporated.
(7) The fibrous film was peeled.
(8) The acicular body was peeled from the intaglio plate.

### [Example 26]

The fibrous sheet of example 25 was replaced with a clean paper (SAKURAI Co., Ltd.; EX Clean Paper EX72BA4T) on which a mold-release treatment was performed with a fluorine-based surface treatment agent (Ryoko Chemicals Co., Ltd.; NOVEC (registered trademark) EGC-1720), and the acicular body was fabricated.

### [Comparative example 4]

The fibrous sheet of example 25 was replaced with a PET film (Toray Industries; Lumirror P60K; thickness of 12 µm), and the acicular body was fabricated.

### [Comparative example 5]

The fibrous sheet of example 25 was replaced with a clean paper (SAKURAI Co., Ltd.; EX Clean Paper EX72BA4T), and the acicular body was fabricated.

### <Measurement of the moisture permeability of an air-permeable film>

Measurement of the moisture permeability of an air-permeable film was conducted by a calcium chloride method, in compliance with "Testing Methods for Water Vapor Permeability of Textiles" JIS-L-1099 (2012).

### (Results)

Fig. 48 shows the measurement results of moisture permeability of the sheet used during pressing and the fabrication results of the acicular body in examples 25 and 26, and comparative examples 4 and 5. In Fig. 48, a circle indicates that an acicular body was fabricated, and a cross indicates that an acicular body could not be fabricated.

In examples 25 and 26, as a result of the acicular bodies being observed under a microscope, it was confirmed that formation was cleanly performed up to the needle tip portion. In comparative example 4, because the moisture permeability of the PET film is low, drying did not progress at all. The acicular body could not be fabricated. In comparative example 5, the clean paper became bonded to and integrated with the acicular body and could not be peeled.

### [Example 27]

A two-layer type acicular body was fabricated by the following method.
(1) First, an acicular-body original plate was formed using precision-machine machining. In the acicular-body original plate, a total of 36 square cones (base: 38 µm × 38 µm; height: 120 µm) are arrayed on a silicon substrate, at 1 mm intervals, in a grid of six rows and six columns.
(2) Next, a mold-release treatment was performed on the acicular-body original plate formed from the silicon substrate, using NOVEC (registered trademark) EGC-1720, a fluorine-based coating agent manufactured by Ryoko Chemicals Co., Ltd.
(3) KU-5550, a urethane for molding manufactured by Hitachi Chemical Co., Ltd., was supplied to the original plate that had been subjected to the mold-release treatment at (2). Curing was performed at 150°C, and an intaglio plate was thereby fabricated.
(4) An aqueous solution to which has been added 20 weight% pullulan (Tokyo Chemical Industry Co., Ltd.) and 1 weight% rhodamine B, as a dye, was dropped onto the intaglio plate fabricated at (3), using a dropper, to an extent that an acicular pattern was covered.
(5) Pressing at a load of 90 N in pressing mode was performed using TENSILON universal material testing machine (A&D Company, Ltd.) to which a cylindrical rod composed of nitrile rubber and having a diameter of 16 mm is attached, over a silicone-coated fibrous sheet (Lion Corporation; REED (registered trademark) Healthy Cooking Sheet / moisture permeability).
(6) The intaglio plate at (5) was dried by being placed on a hot plate set to 90°C for 30 minutes, and moisture was evaporated.
(7) The fibrous film was peeled.
(8) The intaglio plate fabricated at (7) was filled with a 20 weight% aqueous solution of hydroxypropyl cellulose (Tokyo Chemical Industry Co., Ltd.; 6 to 10 mPa·s; 2% in water at 20°C) using a dropper.
(9) The intaglio plate of (5) was dried by being placed on a hot plate set to 40°C for 8 hours, and moisture was evaporated.
(10) The acicular body was peeled from the intaglio plate.

### (Results)

In example 27, as a result of the acicular body being observed under a microscope, it was confirmed that formation was cleanly performed up to the needle tip portion. The appearance of a two-layer type acicular body was confirmed by the coloring.

### [Examples according to the fifth and sixth embodiments]

### [Example 28]

### <Fabricating step for an intaglio plate>

First, an original plate for an acicular body was formed from a silicon substrate using precision-machine machining. The shape of the protrusion portion is a square cone (height: 150 µm; base: 60 µm × 60 µm). On the substrate, 36 protrusion portions are arrayed, at 1 mm intervals, in a grid of six rows and six columns.

Next, a nickel film having a thickness of 500 µm was formed by a plating method on the original plate for an acicular body. Then, the original plate for an acicular body composed of silicon was removed by wet etching with a potassium hydroxide aqueous solution heated to 90°C and having a weight percent concentration of 30%. An intaglio plate composed of nickel was fabricated.

### <Filling step>

Chitosan having a molecular weight of 800,000 was dissolved in an aqueous solution of citric acid, and a liquid body was thereby prepared. The recesses of the intaglio plate was filled with the liquid body using a dispenser, and a filling substance was formed.

### <Drying step>

The intaglio plate filled with the filling substance was disposed on a hot plate set to 90°C and heated for 5 minutes. As a result, the top surface of the liquid body was cured. As a warpage correcting member, an SUS plate having a thickness of 1 cm and in which through-holes of Φ 2 mm are provided at 2 mm intervals was placed in contact with the cured top surface of the liquid body. Heating was further performed for 5 minutes, and drying was completed. As a result, a molded article was formed.

### <Releasing step>

The molded article was peeled from the mold, and an acicular body of example 28 was obtained.

### [Example 29]

### <Fabricating step for an intaglio plate>

An intaglio plate was fabricated by a fabricating step similar to that of example 28.

### <Filling step>

Chitosan having a molecular weight of 800,000 was dissolved in an aqueous solution of citric acid, and a liquid body was thereby prepared. The recesses of the intaglio plate was filled with the liquid body using a dispenser. A chitosan film was further disposed on the top surface of the liquid body, compression was performed, and a filling substance was formed.

### <Drying step>

The intaglio plate filled with the filling substance was disposed on a hot plate set to 90°C and heated for 5 minutes. As a result, the top surface of the chitosan film and the liquid body in contact with the chitosan film was cured. As a warpage correcting member, a screen mesh composed of Tetron (registered trademark), which is composed of polyester resin fibers to which tension is applied, was placed in contact with and disposed on the cured top surface of the filter substance. Heating was further performed for 5 minutes, and drying was completed. As a result, a molded article was formed.

### <Releasing step>

The molded article was peeled from the mold, and an acicular body of example 29 was obtained.

### [Example 30]

### <Fabricating step for an intaglio plate>

An intaglio plate was fabricated by a fabricating step similar to that of example 28.

### <Filling step>

A filling substance composed of a liquid body and a chitosan film was formed by a filling step similar to that of example 29.

### <Drying step>

The intaglio plate filled with the filling substance was disposed on a hot plate set to 90°C and heated for 5 minutes. As a result, the top surface of the chitosan film and the liquid body in contact with the chitosan film was cured. As a warpage correcting member, a fluorine-coated screen mesh composed of Tetron (registered trademark), which is composed of polyester resin fibers to which tension is applied, was placed in contact with and disposed on the cured top surface of the filter substance. Heating was further performed for 5 minutes, and drying was completed. As a result, a molded article was formed.

### <Releasing step>

The molded article was peeled from the mold, and an acicular body of example 30 was obtained.

### [Example 31]

### <Fabricating step for an intaglio plate>

An intaglio plate was fabricated by a fabricating step similar to that of example 28.

### <Filling step>

A filling substance composed of a liquid body and a chitosan film was formed by a filling step similar to that of example 29.

### <Drying step>

The intaglio plate filled with the filling substance was disposed on a hot plate set to 90°C and heated for 5 minutes. As a result, the top surface of the chitosan film and the liquid body in contact with the chitosan film was cured. As a warpage correcting member, a porous film composed of polyethylene was placed in contact with the cured top surface of the filter substance. Heating was further performed for 5 minutes, and drying was completed. As a result, a molded article was formed.

### <Releasing step>

The molded article was peeled from the mold, and an acicular body of example 31 was obtained.

### [Example 32]

### <Fabricating step for an intaglio plate>

An intaglio plate was fabricated by a fabricating step similar to that of example 28.

### <Filling step>

A filling substance composed of a liquid body and a chitosan film was formed by a filling step similar to that of example 29.

### <Drying step>

The intaglio plate filled with the filling substance was disposed on a hot plate set to 90°C and heated for 5 minutes. As a result, the top surface of the chitosan film and the liquid body in contact with the chitosan film was cured. As a warpage correcting member, a screen mesh composed of stainless steel was placed in contact with the cured top surface of the filter substance. Heating was further performed for 5 minutes, and drying was completed. As a result, a molded article was formed.

### <Releasing step>

The molded article was peeled from the mold, and an acicular body of example 32 was obtained.

### [Example 33]

### <Fabricating step for an intaglio plate>

An intaglio plate was fabricated by a fabricating step similar to that of example 28.

### <Filling step>

Chitosan having a molecular weight of 800,000 was dissolved in an aqueous solution of citric acid, and a liquid body was thereby prepared. The recesses of the intaglio plate were filled with the liquid body using a dispenser. A chitosan film was further disposed on the top surface of the liquid body, and a fluorine-coated screen mesh composed of Tetron (registered trademark) was placed in contact with and disposed on the chitosan film. Compression was performed from above the screen mesh, and a filling substance on which the warpage correcting member is placed was formed.

### <Drying step>

In a state where the screen mesh is placed on the filling substance, the intaglio plate filled with the filling substance was disposed on a hot plate set to 90°C and heated for 10 minutes. As a result, a molded article was formed.

### <Releasing step>

The molded article was peeled from the mold, and an acicular body of example 33 was obtained.

### [Example 34]

### <Fabricating step for an intaglio plate>

An intaglio plate was fabricated by a fabricating step similar to that of example 28.

### <Filling step>

A filling substance on which a warpage correcting member is placed was formed by a filling step similar to that of example 33.

### <Drying step>

In a state where the screen mesh is placed on the filling substance, the intaglio plate filled with the filling substance was disposed on a hot plate set to 90°C and heated for 10 minutes. At this time, the screen mesh was separated from the filling substance, and heating was performed in a state where a gap of 200 µm is provided between the chitosan film and the screen mesh. As a result, a molded article was formed.

### <Releasing step>

The molded article was peeled from the mold, and an acicular body of example 34 was obtained.

### [Comparative example 6]

### <Fabricating step for an intaglio plate>

An intaglio plate was fabricated by a fabricating step similar to that of example 28.

### <Filling step>

A filling substance composed of a liquid body was formed by a filling step similar to that of example 28.

### <Drying step>

The intaglio plate filled with the filling substance was disposed on a hot plate set to 90°C and heated for 10 minutes, without disposing a warpage correcting member. As a result, a molded article was formed.

### <Releasing step>

The molded article was peeled from the mold, and an acicular body of comparative example 6 was obtained.

### [Comparative example 7]

### <Fabricating step for an intaglio plate>

An intaglio plate was fabricated by a fabricating step similar to that of example 28.

### <Filling step>

A filling substance composed of a liquid body and a chitosan film was formed by a filling step similar to that of example 29.

### <Drying step>

The intaglio plate filled with the filling substance was disposed on a hot plate set to 90°C and heated for 10 minutes, without using a warpage correcting member. As a result, a molded article was formed.

### <Releasing step>

The molded article was peeled from the mold, and an acicular body of comparative example 7 was obtained.

### [Observation results]

Regarding the acicular bodies of examples 28 to 34 and comparative examples 6 and 7, visual observation was conducted on the back surface of the substrate, which is the surface on the side opposite the surface on which the protrusion portions are formed, such that fluorescent light from the room is caught on the back surface. As a result, no warpage of the acicular body was confirmed in any of examples 28 to 34. Meanwhile, warpage of the substrate was confirmed in comparative examples 6 and 7. Regarding examples 30 to 34, evidence of fiber patterns derived from the warpage correcting member on the back surface of the substrate, resulting from contact with the warpage correcting member, were observed.

The above-described results indicate that the occurrence of warpage in an acicular body is minimized through use of a warpage correcting member, as in examples 28 to 34.

### [Example according to the seventh embodiment]

### [Example 35]

A specific example of the acicular body 1 manufactured using the above-described manufacturing apparatus 110 will be described.

In the present example, an aqueous solution of hydroxypropyl cellulose was used as the liquid body F. After the liquid body F was discharged onto the recess forming surface 22 of the intaglio plate 111, the intaglio plate 111 was conveyed while being vibrated. A wafer paper film was supplied as the film 36. The film 36 was pressed against the recess forming surface 22. The wafer paper film then dissolves in the liquid body F.

Subsequently, moisture was removed in a drying furnace set to 50°C, and the hydroxypropyl cellulose was solidified. Then, the acicular body 1 was obtained by the solidified hydroxypropyl cellulose being released from the intaglio plate 111. Upon examination of the shapes of the protrusion portions 12 in the acicular body 1, it was found that precision of shape was prevented from being impaired due to the mingling of air bubbles.

### [Example according to the eighth embodiment]

### [Example 36]

The acicular body of the present invention was fabricated by the following method.
(1) First, an acicular-body original plate was formed using precision-machine machining. In the acicular-body original plate, a total of 36 square cones (base: 38 µm × 38 µm; height: 120 µm) are arrayed on a silicon substrate, at 1 mm intervals, in a grid of six rows and six columns.
(2) Next, an intaglio plate was fabricated using a heat-curing silicone resin on the acicular-body original plate formed from the silicon substrate.
(3) A chitosan succinamide aqueous solution was prepared as the needle formation liquid.
(4) The obtained intaglio plate was set on the stage. The chitosan succinamide aqueous solution was applied onto the intaglio plate using a smooth nozzle-attached dispenser (nozzle width of 60 mm) such as to cover the overall recesses. At this time, the film thickness is 60 µm.
(5) A porous film (polyethylene material; thickness of 500 µm) was prepared as the film.
(6) The porous film was set on the intaglio plate coated with chitosan succinamide.
(7) A circular cylindrical roll of which the surface is covered with a rubber material was prepared. The diameter of the circular cylindrical roll is 30 mm, and the surface hardness is Shore A 70. Compression was performed while moving the circular cylindrical roll over the surface of the porous film (movement speed of 12.5 mm/sec).
(8) Next, the intaglio plate was dried by being placed on a hot plate set to 90°C for 10 minutes and moisture was evaporated.
(9) The acicular body was peeled from the intaglio plate, and the acicular body was obtained.

### (Results)

As a result of the acicular body obtained in the present example being observed under a microscope, it was confirmed that formation was cleanly performed up to the needle tip.

The embodiments and examples of the present invention are described above. However, the technical scope of the present invention is not limited to the above-described embodiments.

### [Reference Signs List]

- 1: acicular body
- 10: support substrate
- 10a: porous film, film
- 10b: substance liquid
- 11: substrate
- 12: protrusion portion
- 13: liquid body
- 15: support substrate-side layer
- 18: acicular body material liquid
- 20: needle
- 20a: needle forming solution
- 21a: end portion
- 22: recess forming surface
- 23: recess
- 25: needle-side layer
- 25a: needle-side layer forming liquid
- 26: stage
- 26a: setting surface
- 27: conveying direction
- 30: intaglio plate
- 30a, 31: recess
- 35: film covering unit
- 36: film
- 37: feeding device
- 38: intermediate roller
- 39: rotation shaft
- 40: pressing unit
- 41: pressing roller
- 42: platen roller
- 43, 44: rotation shaft
- 45: auxiliary film covering unit
- 46: auxiliary film
- 47: feeding device
- 48: feeding roller
- 50: control unit
- 51: acicular body
- 52: support substrate
- 52a: film
- 53: needle
- 55: cover film covering unit
- 56: cover film
- 57: feeding device
- 60: air-permeable film
- 70: oven
- 110: manufacturing apparatus
- 111: intaglio plate
- 120: warpage correcting member
- 121: air vent
- 125: conveying unit
- 130: liquid body supplying unit
- 131: dispenser
- 151: operating unit
- 152: conveyance amount detector
- 153: tension detector
- 154: tension detector
- 240: table
- 241: coating apparatus
- 242: transferring apparatus
- 243: compressing apparatus
- 250: smooth nozzle-attached dispenser
- 251: film
- 252: film housing body
- 253: suction pad

## Claims

1. A manufacturing method for an acicular body (1; 51) provided with a support substrate (10; 52) and a needle (20; 53) provided on the support substrate (10; 52), the manufacturing method comprising:
a first step of supplying a needle forming solution (20a) onto an intaglio plate (30; 111) having a recess (23; 30a; 31) that has a shape corresponding to the needle (20; 53);
a second step of disposing a film (10a; 36; 52a; 251) directly on the needle forming solution (20a) so that the film gets in contact with the needle forming solution and compressing the needle forming solution (20a) from above the film (10a; 36; 52a; 251) to move the needle forming solution (20a) into the recess (23; 30a; 31); and
a third step of removing liquid components from the needle forming solution (20a) inside the recess (23; 30a; 31) to solidify the needle forming solution (20a), and forming the needle (20; 53).

2. The manufacturing method for an acicular body (1; 51) according to claim 1, wherein the needle forming solution (20a) contains a needle material that forms the needle (20; 53), and the film (10a; 36; 52a; 251) is an air-permeable film (60).

3. The manufacturing method for an acicular body (1; 51) according to claim 1, **characterized in that** the third step includes heating the needle forming solution (20a).

4. The manufacturing method for an acicular body (1; 51) according to claim 1, wherein:
the needle forming solution (20a) is water and the film (10a; 36; 52a; 251) is composed of a needle material; and
the second step includes permitting the water to move into the recess (23; 30a; 31) while dissolving the needle material.

5. The manufacturing method for an acicular body (1; 51) according to claim 4, wherein:
the first step and the second step include permitting the water to attach to the film (10a; 36; 52a; 251) and disposing the film (10a; 36; 52a; 251) on the intaglio plate (30; 111), for supply of water and placement of the film (10a; 36; 52a; 251).

6. The manufacturing method for an acicular body (1; 51) according to claim 1, wherein the needle forming solution (20a) contains a needle material that forms the needle, and the film (10a; 36; 52a; 251) is composed of a water-soluble material.

7. The manufacturing method for an acicular body (1; 51) according to claim 6, wherein the needle material is the same substance as the water-soluble material.

8. The manufacturing method for an acicular body (1; 51) according to any one of claims 1 to 7, wherein the needle forming solution (20a) further contains a substance to be delivered.

9. The manufacturing method for an acicular body (1; 51) according to claim 1, wherein the film (10a; 36; 52a; 251) is a silicone-coated or fluororesin-coated air-permeable film (60).

10. The manufacturing method for an acicular body (1; 51) according to claim 9, wherein
the third step includes a step of forming an acicular body (1; 51) by drying the needle forming solution (20a) in a state where the air-permeable film (60) is attached to the needle forming solution (20a) that has been moved into the recess (23; 30a; 31); and
the manufacturing method further includes a step of peeling the air-permeable film (60) from the acicular body (1; 51).

11. The manufacturing method for an acicular body (1; 51) according to claim 10, further comprising:
a step of supplying additional needle forming solution (20a) to the intaglio plate (30; 111) after peeling of the air-permeable film (60); and
a step of drying the additional needle forming solution (20a), followed by separating the acicular body (1; 51) from the intaglio plate (30; 111).

## Patentansprüche

1. Verfahren zur Herstellung eines nadelförmigen Körpers (1; 51), der mit einem Trägersubstrat (10; 52) und einer auf dem Trägersubstrat (10; 52) bereitgestellten Nadel (20; 53) versehen ist, wobei das Herstellungsverfahren umfasst:
einen ersten Schritt des Zuführens einer Nadelbildungslösung (20a) auf eine Tiefdruckplatte (30; 111) mit einer Aussparung (23; 30a; 31), die eine der Nadel (20; 53) entsprechende Form aufweist;
einen zweiten Schritt des Anordnens einer Folie (10a; 36; 52a; 251) direkt auf der Nadelbildungslösung (20a), so dass die Folie mit der Nadelbildungslösung in Kontakt kommt, und des Komprimierens der Nadelbildungslösung (20a) von oberhalb der Folie (10a; 36; 52a; 251), um die Nadelbildungslösung (20a) in die Aussparung (23; 30a; 31) zu bewegen; und
einen dritten Schritt des Entfernens von flüssigen Komponenten aus der Nadelformlösung (20a) innerhalb der Vertiefung (23; 30a; 31), um die Nadelformlösung (20a) zu verfestigen, und des Formens der Nadel (20; 53).

2. Verfahren zur Herstellung eines nadelförmigen Körpers (1; 51) nach Anspruch 1, wobei die Nadelbildungslösung (20a) ein Nadelmaterial enthält, das die Nadel (20; 53) bildet, und die Folie (10a; 36; 52a; 251) eine luftdurchlässige Folie (60) ist.

3. Verfahren zur Herstellung eines nadelförmigen Körpers (1; 51) nach Anspruch 1, **dadurch gekennzeichnet, dass** der dritte Schritt das Erwärmen der Nadelbildungslösung (20a) beinhaltet.

4. Verfahren zur Herstellung eines nadelförmigen Körpers (1; 51) nach Anspruch 1, wobei:
die Nadelbildungslösung (20a) Wasser ist und die Folie (10a; 36; 52a; 251) aus einem Nadelmaterial besteht; und
der zweite Schritt beinhaltet, dass das Wasser in die Vertiefung (23; 30a; 31) eindringen kann, während das Nadelmaterial aufgelöst wird.

5. Verfahren zur Herstellung eines nadelförmigen Körpers (1; 51) nach Anspruch 4, wobei:
der erste Schritt und der zweite Schritt beinhalten das Zulassen, dass sich das Wasser an die Folie (10a; 36; 52a; 251) anhaftet, und das Anordnen der Folie (10a; 36; 52a; 251) auf der Tiefdruckplatte (30; 111), um Wasser zuzuführen und die Folie (10a; 36; 52a; 251) anzuordnen.

6. Verfahren zur Herstellung eines nadelförmigen Körpers (1; 51) nach Anspruch 1, wobei die Nadelbildungslösung (20a) ein Nadelmaterial enthält, das die Nadel bildet, und die Folie (10a; 36; 52a; 251) aus einem wasserlöslichen Material besteht.

7. Verfahren zur Herstellung eines nadelförmigen Körpers (1; 51) nach Anspruch 6, wobei das Nadelmaterial die gleiche Substanz wie das wasserlösliche Material ist.

8. Verfahren zur Herstellung eines nadelförmigen Körpers (1; 51) nach einem der Ansprüche 1 bis 7, wobei die Nadelbildungslösung (20a) ferner eine abzugebende Substanz enthält.

9. Verfahren zur Herstellung eines nadelförmigen Körpers (1; 51) nach einem der Ansprüche 1, wobei die Folie (10a; 36; 52a; 251) eine luftdurchlässige Folie (60) ist, die mit Silicon oder Fluorharz beschichtet ist.

10. Verfahren zur Herstellung eines nadelförmigen Körpers (1; 51) nach Anspruch 9, wobei
der dritte Schritt einen Schritt des Bildens eines nadelförmigen Körpers (1; 51) durch Trocknen der Nadelbildungslösung (20a) in einem Zustand beinhaltet, in dem die luftdurchlässige Folie (60) an der Nadelbildungslösung (20a) angehaftet ist, die in die Vertiefung (23; 30a; 31) bewegt wurde; und
das Herstellungsverfahren ferner einen Schritt des Ablösens der luftdurchlässigen Folie (60) vom nadelförmigen Körper (1; 51) beinhaltet.

11. Das Herstellungsverfahren für einen nadelförmigen Körper (1; 51) nach Anspruch 10 umfasst ferner:
einen Schritt des Zuführens zusätzlicher Nadelbildungslösung (20a) zu der Tiefdruckplatte (30; 111) nach dem Ablösen der luftdurchlässigen Folie (60); und
einen Schritt des Trocknens der zusätzlichen Nadelbildungslösung (20a), gefolgt von der Trennung des nadelförmigen Körpers (1; 51) von der Tiefdruckplatte (30; 111).

## Revendications

1. Procédé de fabrication pour un corps aciculaire (1 ; 51) prévu avec un substrat de support (10 ; 52) et une aiguille (20 ; 53) prévue sur le substrat de support (10 ; 52), le procédé de fabrication comprenant :
une première étape pour fournir une solution de formage d'aiguille (20a) sur une plaque de gravure en creux (30 ; 111) ayant un évidement (23 ; 30a ; 31) qui a une forme correspondant à l'aiguille (20 ; 53) ;
une deuxième étape pour disposer un film (10a ; 36 ; 52a ; 251) directement sur la solution de formage d'aiguille (20a) de sorte que le film vient en contact avec la solution de formage d'aiguille et comprimant la solution de formage d'aiguille (20a) depuis le dessus du film (10a ; 36 ; 52a ; 251) pour déplacer la solution de formage d'aiguille (20a) dans l'évidement (23 ; 30a ; 31) ; et
une troisième étape pour retirer les composants liquides de la solution de formage d'aiguille (20a) à l'intérieur de l'évidement (23 ; 30a ; 31) afin de solidifier la solution de formage d'aiguille (20a) et former l'aiguille (20 ; 53).

2. Procédé de fabrication pour un corps aciculaire (1 ; 51) selon la revendication 1, dans lequel la solution de formage d'aiguille (20a) contient un matériau d'aiguille qui forme l'aiguille (20 ; 53), et le film (10a ; 36 ; 52a ; 251) est un film perméable à l'air (60).

3. Procédé de fabrication pour un corps aciculaire (1 ; 51) selon la revendication 1, **caractérisé en ce que** la troisième étape comprend l'étape pour chauffer la solution de formage d'aiguille (20a).

4. Procédé de fabrication pour un corps aciculaire (1 ; 51) selon la revendication 1, dans lequel :
la solution de formage d'aiguille (20a) est de l'eau et le film (10a ; 36 ; 52a ; 251) est composé d'un matériau d'aiguille ; et
la deuxième étape comprend l'étape pour permettre à l'eau de se déplacer dans l'évidement (23 ; 30a ; 31) tout en dissolvant le matériau d'aiguille.

5. Procédé de fabrication pour un corps aciculaire (1 ; 51) selon la revendication 4, dans lequel :
la première étape et la deuxième étape comprennent les étapes pour permettre à l'eau à se fixer sur le film (10a ; 36 ; 52a ; 251) et déposer le film (10a ; 36 ; 52a ; 251) sur la plaque de gravure en creux (30 ; 111), pour la fourniture de l'eau et la mise en place du film (10a ; 36 ; 52a ; 251).

6. Procédé de fabrication pour un corps aciculaire (1 ; 51) selon la revendication 1, dans lequel la solution de formage d'aiguille (20a) contient un matériau d'aiguille qui forme l'aiguille, et le film (10a ; 36 ; 52a ; 251) est composé d'un matériau soluble dans l'eau.

7. Procédé de fabrication pour un corps aciculaire (1 ; 51) selon la revendication 6, dans lequel le matériau d'aiguille est la même substance que le matériau soluble dans l'eau.

8. Procédé de fabrication pour un corps aciculaire (1 ; 51) selon l'une quelconque des revendications 1 à 7, dans lequel la solution de formage d'aiguille (20a) contient en outre une substance à distribuer.

9. Procédé de fabrication pour un corps aciculaire (1 ; 51) selon la revendication 1, dans lequel le film (10a ; 36 ; 52a ; 251) est un film perméable à l'air recouvert de silicone ou recouvert de résine fluorée (60).

10. Procédé de fabrication pour un corps aciculaire (1 ; 51) selon la revendication 9, dans lequel :
la troisième étape comprend une étape pour former un corps aciculaire (1 ; 51) en faisant sécher la solution de formage d'aiguille (20a) dans un état dans lequel le film perméable à l'air (60) est fixé sur la solution de formage d'aiguille (20a) qui a été déplacée dans l'évidement (23 ; 30a ; 31) ; et
le procédé de fabrication comprend en outre une étape pour détacher le film perméable à l'air (60) du corps aciculaire (1 ; 51).

11. Procédé de fabrication pour un corps aciculaire (1 ; 51) selon la revendication 10, comprenant en outre :
une étape pour amener la solution de formage d'aiguille supplémentaire (20a) à la plaque de gravure en creux (30 ; 111) après le détachement du film perméable à l'air (60) ; et
une étape pour faire sécher la solution de formage d'aiguille supplémentaire (20a), suivie par l'étape pour séparer le corps aciculaire (1 ; 51) de la plaque de gravure en creux (30 ; 111).
